# EUROPEAN PATENT APPLICATION

(11) **EP 4 571 782 A1**
(43) Date of publication of application: **18.06.2025**
(21) Application number: 23852030.8
(22) Date of filing: 14.08.2023
(51) Int. Cl.: G16H 80/00

(54) **LIFE INFORMATION PROCESSING SYSTEM AND LIFE INFORMATION PROCESSING METHOD**

(30) Priority: 12.08.2022 CN 202210970377; 02.06.2023 WO PCT/CN2023/098103; 02.06.2023 WO PCT/CN2023/098138; 02.06.2023 WO PCT/CN2023/098116
(71) Applicant: Shenzhen Mindray Bio-Medical Electronics Co., Ltd., Shenzhen, Guangdong 518057 (CN)
(72) Inventor: HE, Xianliang, Shenzhen, Guangdong 518057 (CN); GUAN, Zehong, Shenzhen, Guangdong 518057 (CN); JIN, Xingliang, Shenzhen, Guangdong 518057 (CN); XIAO, Ke, Shenzhen, Guangdong 518057 (CN); LIU, Sanchao, Shenzhen, Guangdong 518057 (CN); YU, Zedan, Shenzhen, Guangdong 518057 (CN); SUN, Bailei, Shenzhen, Guangdong 518057 (CN); YE, Wenyu, Shenzhen, Guangdong 518057 (CN); LI, Ming, Shenzhen, Guangdong 518057 (CN); XU, Li, Shenzhen, Guangdong 518057 (CN); CEN, Jian, Shenzhen, Guangdong 518057 (CN)
(74) Representative: KIPA AB
(86) International application number: PCT/CN2023/112990
(87) International publication number: WO 2024/032811

(57) **Abstract**

A life information processing system and a life information processing method. The system comprises a memory, a processor, and a display. The processor is configured to execute the following operations: obtaining multiple types of patient data of a patient; analyzing the patient data to determine a patient status of the patient; analyzing the patient data to determine one or more target rules satisfied by the patient data; and controlling the display to display patient status prompt information representing the patient status and to display at least part of the one or more target rules. The system provides explanatory description while the patient status is outputted, can improve the credibility of the patient status, and helps medical staff to perform better diagnosis or treatment.

## Description

### TECHNICAL FIELD

The disclosure relates to a technical field of vital information, and more particularly to a system for processing vital information and a method for processing vital information.

### BACKGROUND

With the development of monitoring technology, a current monitoring device is already able to monitor, in real time, important vital sign parameters, such as an ECG parameter, a blood pressure parameter, a blood oxygen saturation parameter, a respiratory rate parameter, a body temperature parameter, etc., provide important patient information for medical clinical diagnosis and conduct supervision and alarm for each parameter, based on signals of high-precision and high-sampling rate for these vital sign parameters.

Traditional monitoring strategies mainly focus on monitoring a physiological parameter, that is, obtaining a physiological signal, extracting a vital sign parameter based on the physiological signal, and then determining whether the parameter exceeds a preset threshold. If the threshold is exceeded, an audible, visual, and graph alarm is issued to remind medical staff that a certain specific vital sign value of a current specific patient exceeds a standard. The above analysis method does not effectively utilize a large amount of vital sign parameter information provided by the currently fully developed monitoring technology, and cannot provide intuitive and convenient indication for change(s) in a condition of the patient. The above analysis method is only an objective description of the physiological parameter of the patient and is highly dependent on experience of doctor in interpreting the vital sign parameter. Doctor with relatively limited qualification is prone to ignore worsening of medical condition implied by a simultaneous change in multiple parameters, thus delaying diagnosis and treatment of the patient.

### SUMMARY

A series of simplified concepts are introduced into summary of this disclosure, and are further elaborated in specific embodiments of this disclosure. The summary of this disclosure does not attempt to define key and necessary technical characteristics of the claimed technical solution, nor attempt to determine a protection scope of the claimed technical solution.

A first aspect according to an embodiment of this disclosure provides a system for processing vital information, including a memory, a processor, and a display; wherein the memory is configured to store an executable program, and the processor is configured to execute the executable program, so as to enable the processor to perform following operations:
obtaining multiple types of patient data of a patient;
analyzing the patient data, so as to determine one or more target rules which are satisfied by the patient data;
determining a patient state of the patient which corresponds to the one or more target rules, wherein the patient state at least includes a state of a physiological system of the patient; the physiological system of the patient includes at least one of a nervous system, a circulatory system, and a respiratory system, of the patient; and
controlling the display to display indication information which indicates the patient state, and to display at least a portion of the one or more target rules; wherein the indication information which indicates the patient state at least includes an overall assessment of the state of the physiological system of the patient; wherein
when the physiological system of the patient includes the nervous system of the patient, the one or more target rules include an indicator which is related to a cranial nerve;
when the physiological system of the patient includes the circulatory system of the patient, the one or more target rules include an indicator which is related to hemodynamics or perfusion;
when the physiological system of the patient includes the respiratory system of the patient, the one or more target rules include an indicator which is related to oxygenation.

A second aspect according to an embodiment of this disclosure provides a method for processing vital information, including:
obtaining multiple types of patient data of a patient;
analyzing the patient data, so as to determine a patient state of the patient, wherein the patient state at least includes a state of a physiological system of the patient; the physiological system includes at least one of a nervous system, a circulatory system, and a respiratory system, of the patient;
analyzing the patient data, so as to determine one or more target rules which are satisfied by the patient data;
displaying indication information which indicates the patient state, and displaying at least a portion of the one or more target rules; wherein the indication information which indicates the patient state at least includes an overall assessment of the state of the physiological system of the patient; wherein
when the physiological system of the patient includes the nervous system of the patient, the one or more target rules include an indicator which is related to a cranial nerve;
when the physiological system of the patient includes the circulatory system of the patient, the one or more target rules include an indicator which is related to hemodynamics or perfusion;
when the physiological system of the patient includes the respiratory system of the patient, the one or more target rules include an indicator which is related to oxygenation.

A third aspect according to an embodiment of this disclosure provides a method for processing vital information, including:
obtaining multiple types of patient data of a patient;
analyzing the patient data, so as to determine a patient state of the patient and to determine at least a portion of information which is extracted from the patient data and used to determine the patient state; wherein the patient state at least includes a state of a physiological system of the patient; the physiological system includes at least one of a nervous system, a circulatory system, and a respiratory system, of the patient;
displaying indication information which indicates the patient state, and displaying at least a portion of information which is extracted from the patient data; wherein the indication information which indicates the patient state at least includes an overall assessment of the state of the physiological system of the patient; wherein
when the physiological system of the patient includes the nervous system of the patient, the information which is extracted from the patient data includes an indicator which is related to a cranial nerve;
when the physiological system of the patient includes the circulatory system of the patient, the information which is extracted from the patient data includes an indicator which is related to hemodynamics or perfusion;
when the physiological system of the patient includes the respiratory system of the patient, the information which is extracted from the patient data includes an indicator which is related to oxygenation.

A fourth aspect according to an embodiment of this disclosure provides a method for processing vital information, including:
obtaining multiple types of patient data of a patient;
analyzing the patient data, so as to determine a patient state of the patient;
analyzing the patient data, so as to determine one or more target rules which are satisfied by the patient data;
displaying indication information which indicates the patient state, and displaying at least a portion of the one or more target rules.

According to the system for processing vital information and the method for processing vital information according to an embodiment of this disclosure, by comprehensively analyzing multiple types of patient data to obtain a patient state, a correlation between multiple types of patient data is fully considered, which can avoid problems, such as false alarm and missed alarm, and can help medical staff to quickly and reliably determine a medical condition of the patient, which is conducive to real-time grasp of a health state of the patient. Furthermore, when outputting a patient state, target rule(s) is(are) also displayed, which provides explanatory explanations for the patient state, enhances a credibility of the patient state, and assists medical staff to diagnose or treat the patient better.

### BRIEF DESCRIPTION OF THE DRAWINGS

In order to provide a clearer explanation of technical solutions in embodiments of this disclosure, a brief introduction is given to the accompanying drawings required in the description of the embodiments. It is evident that the accompanying drawings in the following description are only some embodiments of this disclosure. For ordinary technical personnel in the art, other accompanying drawings can be obtained based on these drawings without any creative effort.

Wherein:
FIG. 1 is a block diagram of a system for processing vital information according to an embodiment of this disclosure.
FIG. 2 is a diagram of a display interface according to an embodiment of this disclosure.
FIG. 3 is a flow chart of a method for processing vital information according to an embodiment of this disclosure.
FIG. 4 is a flow chart of a method for processing vital information according to another embodiment of this disclosure.
FIG. 5 is a diagram of a display interface according to another embodiment of this disclosure.
FIG. 6 is a diagram of a display interface according to a further embodiment of this disclosure.

### DESCRIPTION OF THE PREFERRED EMBODIMENTS

In order to make the purpose, technical solution, and advantages of this disclosure more obvious, the following refers to the attached drawings to describe in detail the exemplary embodiments according to this disclosure. Obviously, the described embodiments are only some of the embodiments of this disclosure, not all of them. It should be understood that this disclosure is not limited by the embodiments described here. Based on the embodiments described in this disclosure, all other embodiments obtained by those skilled in the art without creative work, fall within the protection scope of this disclosure.

In the following description, a large number of specific details are provided to provide a more thorough understanding of this disclosure. However, it is evident to those skilled in the art that this disclosure can be implemented without the need for one or more of these details. In other examples, in order to avoid confusion with this disclosure, some well-known technical characteristics in this field are not described.

It should be understood that this disclosure can be implemented in different forms and should not be limited to the embodiments proposed here. On the contrary, providing these embodiments make the disclosure thorough and complete, and fully convey the scope of this disclosure to those skilled in the art.

The purpose of the terminology used here is only to describe specific embodiments and is not a limitation of this disclosure. When used here, the singular forms of "an", "a", and "the/said" are also intended to include the plural form, unless the context clearly indicates another way. It should also be understood that the terms, such as "including", "and/or", "comprising", when used in this disclosure, determine the presence of the characteristics, integers, steps, operations, components, and/or elements, but do not exclude the presence or addition of one or more other characteristics, integers, steps, operations, components, and/or elements. When used here, the term "and/or" includes any and all combinations of related listed items. In addition, the disclosure can be implemented in various forms and is not limited to the embodiments described in this embodiment. The purpose of providing the following specific embodiments is to facilitate a clearer and more thorough understanding of the disclosed content of this application, wherein words indicating directions such as up, down, left, and right are only for positions of the structures shown in the corresponding drawings. The term "display interface" may be an interface of a system for processing vital information that displays parameter waveform(s) and/or parameter values(s); or may be an interface displayed after the system for processing vital information is turned on; or be an interface that is used more frequently by the system for processing vital information.

In order to fully understand this disclosure, a detailed structure is provided in the following description to explain the technical solution proposed in this disclosure. The optional embodiments of this disclosure are described in detail below, however, in addition to these detailed description, this disclosure may also have other embodiments.

At present, a monitoring device mainly uses following alarm methods for alarming, a first alarm method based on data of single vital sign, which alarms when a value of data of single vital sign is determined to exceed an alarm threshold at a preset time point or within a preset period of time; a second alarm method based on multiple vital sign data, which alarms when multiple vital sign data simultaneously exceeds alarm thresholds; a third alarm method based on a trend of data of single vital sign, which alarms when data of single vital sign is determined to show a downtrend or uptrend within a preset period of time.

However, there is usually a correlation between vital sign data and other patient data, and a patient state is often reflected in a comprehensive performance of multiple types of patient data. The alarm method of existing monitoring device does not take this correlation into consideration, which is prone to problems, such as false alarms and missed alarms. In addition, the existing monitoring device can only provide an objective description of a physiological state of the patient. The interpretation on vital sign parameter(s) depends largely on experience of doctor. Doctor with relatively limited qualification is prone to ignore worsening of medical condition implied by a simultaneous change in multiple parameters, thus delaying diagnosis and treatment of the patient. Individual parameter monitoring cannot effectively utilize the large amount of vital sign parameter information acquired by the monitoring device, and cannot find changes in the patient state which changes are conveyed by the vital sign parameters.

In addition, although there are related methods that conduct comprehensive analysis of multiple vital sign parameters based on machine learning and other technologies, due to the current shortcomings of machine learning and other technologies, it is easy to output abnormal alarms that are difficult to be accepted clinically. Moreover, such methods are generally highly dependent on training data. In the current situation where, clinical data is difficult to obtain, it is even more difficult to conduct sufficient model training, and the results of the model output are difficult to gain the trust of clinical medical staff.

In view of the above-mentioned deficiencies of the existing monitoring device, an embodiment of this disclosure provides a system for processing vital information to implement a state monitoring. State monitoring refers to monitoring a patient state and providing an indication or alarm when an abnormal patient state is detected. The patient state is an assessment for overall or local physiological function of the patient, and reflects overall or local health state of the patient. Furthermore, in the system for processing vital information of an embodiment of this disclosure, when outputting a patient state, is capable of providing explanatory explanation for the patient state, so as to explain a specific reason for the current patient state, thus improving a credibility of the patient state, and assisting medical staff in quickly grasping a patient situation.

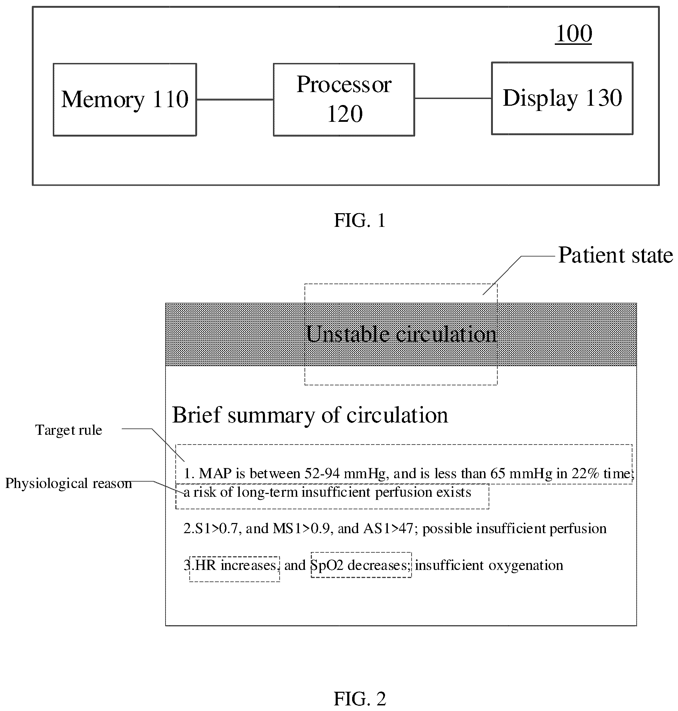

Referring to FIG. 1, the system for processing vital information 100 of an embodiment of this disclosure includes a memory 110, a processor 120 and a display 130, wherein the memory 110 is configured to store an executable program, and the processor 120 is configured to execute the executable program stored in the memory 110, so as to enable the processor 120 to perform following operations: obtaining multiple types of patient data of a patient; analyzing the patient data, so as to determine one or more target rules which are satisfied by the patient data; determine a patient state of the patient which corresponds to the one or more target rules; and controlling the display 130 to display indication information which indicates the patient state, and to display at least a portion of the one or more target rules.

The system for processing vital information of the embodiment of this disclosure obtains a patient state by comprehensive analysis of multiple types of patient data, and displays patient-related information. Compared with the monitoring plan based on a parameter value or a parameter trend, the monitoring plan based on the patient state fully considers the correlation between various types of patient data, and can avoid problems such as false alarms and missed alarms in the system for processing vital information. Medical staff can quickly and reliably determine the medical condition of the patient based on patient state information, which is conducive to real-time grasp of a health state of the patient.

The system for processing vital information 100 of the embodiment of this disclosure includes but is not limited to any one or a combination of: a monitor, a local central station, a remote central station, a cloud service system, and a mobile terminal. The system for processing vital information 100 may be a portable system for processing vital information, a transportable system for processing vital information, or a mobile system for processing vital information, etc.

In one embodiment, the system for processing vital information 100 may be a monitor, which is configured to monitor monitoring parameter(s) of patient(s) in real time. The monitor may include a bedside monitor, a wearable monitor, and the likes. The monitor may further include a ventilator monitor, an anesthesia monitor, a defibrillation monitor, an intracranial pressure monitor, an ECG monitor, etc.

The system for processing vital information 100 may also include a central station for receiving monitoring data which is transmitted by the monitor and performing centralized monitoring of the monitoring data. The central station may include a local central station or a remote central station. The central station is connected with monitor(s) in one or more departments through a network to achieve real-time centralized monitoring and massive data storage. For example, the central station stores, but is not limited to store, monitoring data, basic patient information data, medical history information, and diagnostic information.

In some embodiments, the monitor and the central station can form an interconnection platform through BeneLink to achieve data communication between the monitor and the central station. For example, the central station can access the monitoring data detected by the monitor. In some other embodiments, the monitor and the central station can also establish a data connection through a communication unit, which includes but is not limited to, Wi-Fi, Bluetooth or mobile communication 2G, 3G, 4G, 5G and other communication units.

The system for processing vital information 100 of the embodiment of this disclosure can also include other device(s) besides a monitoring device, such as, an image acquisition device, a treatment and support device, an information system (such as CIS, HIS, handover software, decision support system, etc.), a mobile terminal (such as ward round vehicle), etc.

The processor 120 of the system for processing vital information 100 can be a central processing unit (CPU), or another general-purpose processor, a digital signal processor (DSP), an application-specific integrated circuit (ASIC), a field-programmable gate array (FPGA), or another programmable logic device, a discrete gate or a transistor logic device, a discrete hardware components etc. A general purpose processor may be a microprocessor, or any conventional processor or the likes. The processor 120 is a control center of the system for processing vital information 100, and uses various interfaces and lines to connect various parts of the entire system for processing vital information 100.

The memory 110 of the system for processing vital information 100 is configured to store an executable program. Furthermore, the memory 110 may also store patient data, such as vital sign data of a patient, which patient is associated with the system for processing vital information 100. Exemplarily, the memory 110 may mainly include a program storage area and a data storage area, wherein the program storage area may store an operating system, application programs which are required for multiple functions, and the like. In addition, the memory 110 may include a high-speed random access memory and may also include a non-volatile memory, such as a hard disk, a memory, a plug-in hard disk, a smart memory card, a secure digital card, a flash memory card, multiple disk storage devices, a flash memory device, or another volatile solid-state storage device.

The display 130 is configured to provide a user with a visual output. Specifically, the display 130 can be configured to provide a visual display interface for a user, including but not limited to, a monitoring interface, an interface for setting monitoring parameter(s), etc. Exemplarily, the display 130 may be implemented as a touch display, or the display 130 may have an input panel, that is, the display 130 may serve as an input/output device.

In some embodiments, the system for processing vital information 100 further includes a data acquisition unit, such as a sensor. The sensor can be used to continuously acquire monitoring data of patient(s). Wherein, continuously acquiring means that the sensor continuously measures the monitoring data for multiple times at preset time intervals, and the preset time interval refers to a shortest time length which is needed by the sensor to return the monitoring data. The data acquisition unit and the processor 120 may be connected via a wired communication protocol or a wireless communication protocol, so that data exchange can be performed between the data acquisition unit and the processor 120. Wireless communication technologies include but are not limited to various generations of mobile communication technologies (2G, 3G, 4G and 5G), a wireless network, Bluetooth, ZigBee, ultra-wideband UWB, NFC, etc.

Specifically, the data acquisition unit is configured to acquire vital sign data of the patient. In some embodiments, the data acquisition unit may be independently disposed outside the system for processing vital information 100 and detachably connected with the system for processing vital information 100. The processor 120 is further configured to process the vital sign data from the data acquisition unit. In some embodiments, the system for processing vital information 100 may not include a sensor, and the system for processing vital information 100 may receive monitoring data which is acquired by an external monitoring accessory through a communication unit.

The system for processing vital information 100 may further include a communication unit which is connected with the processor 120. In some embodiments, the system for processing vital information 100 can establish data communication with a third-party device through the communication unit. The processor 120 further controls the communication unit to acquire data from a third-party device, or transmits the vital sign data which are acquired by the data acquisition unit to a third-party device. The communication unit includes but is not limited to Wi-Fi, Bluetooth, NFC, ZigBee, ultra-wideband UWB or 2G, 3G, 4G, 5G and other mobile communication units. In some other embodiments, the system for processing vital information 100 may also establish a connection with a third-party device via a cable. Third-party device includes but is not limited to a ventilator, an anesthesia machine, an infusion pump, an image acquisition device and other medical devices. Third-party devices can also be a cloud service system or a non-medical device, such as a mobile phone, a tablet PC, and a personal computer.

Compared with the system for processing vital information that performs a parameter alarm, the system for processing vital information 100 of the embodiment of this disclosure performs a state monitoring. Compared with the parameter alarm, state monitoring is more complex clinically, and vital sign data alone is difficult to support determination of a patient state. Therefore, the system for processing vital information 100 of the embodiment of this disclosure obtains multiple types of patient data through multiple channels such as a data acquisition unit and a communication unit, and performs a comprehensive analysis on the multiple types of patient data, so that the patient state obtained by this analysis is more accurate, avoiding obtaining incorrect patient state or being unable to obtain the patient state due to insufficient information.

In some embodiments, the system for processing vital information 100 further includes an alarm unit which is connected with the processor 120 for outputting an alarm indication so that medical personnel can perform corresponding rescue measures. The alarm unit includes but is not limited to an alarm light, an alarm speaker, etc. Alarm information may also be displayed on the display 130, an alarm light may flash to alert medical personnel, or an alarm speaker may play the alarm information.

In order to realize user interface and data exchange, in addition to the display 130, the system for processing vital information 100 may also include other input/output device(s) which is(are) connected with the processor 120, including but not limited to, an input device such as a keyboard, a mouse, a touch screen, a remote control, and an output device including but not limited to a printer, a speaker, etc.

It should be understood that FIG. 1 is merely an example of components included in the system for processing vital information 100 and does not constitute a limitation on the system for processing vital information 100, and the system for processing vital information 100 may include more or fewer components, or a combination of certain components, or different components, than that shown in FIG. 1.

During providing a monitoring for the patient via the system for processing vital information 100, the processor 120 obtains multiple types of patient data of the patient to fully determine the patient state. The patient state may be at least one of: an overall state of the patient, a state of a physiological system of the patient, a state of an organ of the patient, a state of a physiological part of the patient, a state of a tissue of the patient. Since a nervous system, a respiratory system, and a circulatory system of the patient are most clinically concerned, the state of the physiological system may include a state of the nervous system, a state of the respiratory system, and a state of the circulatory system. The state of a system, organ, or tissue reflects a macroscopic state of the corresponding system, organ, or tissue.

Exemplarily, the patient data includes at least one or more vital sign data of the patient. Wherein vital sign data includes but is not limited to at least one of: electrocardiogram data, blood pressure data, pulse oximetry data, respiration data, body temperature data, cardiac output data, carbon dioxide data, movement data, video data, respiratory mechanics parameter data, hemodynamic parameter data, oxygen metabolism parameter data, electroencephalogram parameter data, bi-spectral index data and microcirculation parameter data. The processor 120 may obtain the vital sign data which data is acquired by the system for processing vital information 100 itself from the data acquisition unit, or may receive the vital sign data of the patient from an external device through the communication unit.

The vital sign data which is acquired by the processor 120, may be high-precision, high-sampling-frequency vital sign data which is related to the patient who is monitored by the system for processing vital information 100, such as real-time monitoring data, such as electrocardiogram data, blood pressure data, respiration data, blood oxygen data, body temperature data, and cardiac output data. Such data changes rapidly over time and is acquired at a high frequency, and can be presented in real time on a monitoring interface of the system for processing vital information 100. Due to different acquisition conditions, a sampling frequency of vital sign data may vary. For example, blood pressure data is divided into non-invasive blood pressure data and invasive blood pressure data. Invasive blood pressure data is real-time monitoring data, while non-invasive blood pressure data is generally intermittent measurement data. For example, a non-invasive blood pressure is measured every 30 minutes to obtain systolic data, diastolic data and average blood pressure data of a patient. Similar intermittent measurement data also include body temperature data, urine volume data, blood sugar data and other data.

The processor 120 may acquire the vital sign data in various ways. For example, one way is to acquire the vital sign data of real-time monitoring, and another way is to acquire the vital sign data during a monitoring period for the patient state at one time. In addition to the vital sign data obtained by the system for processing vital information 100 itself, the system for processing vital information 100 can also obtain other patient data from external medical device(s) or non-medical device(s). By way of example, an external device includes a treatment device, a test device, and a third-party system, among which the treatment device includes a ventilator, an anesthesia machine, an infusion pump, an extracorporeal circulation device, etc., a test device includes an ultrasound imaging device, an endoscopic device, etc., and a third-party system includes a PACS system (picture archiving and communication system), an LIS system (laboratory information system), a CIS system (clinical information system), etc. Exemplarily, the patient data obtained from the external device includes at least one of monitoring data and/or device data which is acquired by the ventilator device, monitoring data and/or device data which is acquired by the anesthesia machine, monitoring data and/or device data which is acquired by the infusion pump device, medical condition data, test data, and examination data. The system for processing vital information 100 can obtain the above patient data through a communication connection with an external device.

Exemplarily, the medical condition data includes at least one of: basic patient information data, disease diagnosis data, treatment data, nursing data, and electronic medical record data. Wherein, the basic patient information includes age, weight, gender, etc., and the disease diagnosis data includes a medical history, a diagnosis report, a medical advice, a consultation conversation, etc. The system for processing vital information 100 can obtain medical condition data of the patient through an electronic medical record, or receive the medical condition data inputted by medical staff. The test data includes at least one of: routine blood test data, liver function test data, kidney function test data, thyroid test data, urine test data, immune test data, coagulation test data, blood gas test data, routine stool test data, tumor marker test data, which is(are) acquired by in-vitro diagnostic device(s). The system for processing vital information 100 can obtain the examination data of the patient through an information management system of a hospital laboratory. The examination data includes data, which are acquired by a medical imaging device and specifically include at least one of: DR imaging data, CT imaging data, MRI imaging data, PET imaging data, ultrasound imaging data, scale data, and physical examination data. The system for processing vital information 100 can obtain the examination data from the medical imaging device or an image summarization and communication system.

The patient data acquired by the processor 120 may be medical data within a certain time range, which range may be a preset time range, such as 24 hours, or a suitable time range which is set according to a user instruction. Specifically, patient data within a certain time range may be selected according to a timestamp of the medical data. If certain types of patient data do not exist within this time range, for example, certain laboratory examination data may not have examination results within 24 hours, most recently acquired patient data of this type may be selected for subsequent data processing.

For some patient data, especially physiological data which is acquired by the data acquisition device of the system for processing vital information 100, the processor 120 may also perform preprocessing, including filtering, abnormal/null value processing, data sampling alignment and other operations. Data sampling alignment refers to interpolation operation of intermittently measured data, such as blood pressure data, for ensuring that the intermittently measured data can be analyzed synchronously with other data.

By preprocessing the patient data, patient data that satisfy a quality requirement can be extracted for subsequent analysis, especially for continuous measurement data in the patient data. Performing quality analysis on these data helps to filter out data that is generated by interference and has no actual clinical value. The above-mentioned patient data acquired by the system for processing vital information 100 can present various information about the patient, but it can also be seen from the relevant description that the patient data related to the patient are diverse, and a large part of the data changes over time. It is difficult for clinical medical staff to conduct comprehensive and efficient clinical assessment and decision-making based on the above-mentioned patient data. Therefore, the system for processing vital information 100 of the embodiment of this disclosure automatically determines the patient state according to the patient data, so as to make full use of the patient data and quickly extract valid information from the patient data.

Exemplarily, in order to determine the patient state, the processor 120 may determine a target rule which is satisfied by the patient data, thereby determining the patient state according to a corresponding relationship between the target rule and the patient state. Since data types and data volumes of the patient data are numerous, the patient data may be first analyzed to obtain one or more analysis results which reflect characteristic(s) of the patient data, and the target rule which is satisfied by the patient data may be determined based on the analysis result(s). The analysis result(s) can be quantitative feature(s) or non-quantitative feature(s) extracted from the patient data. Exemplarily, an analysis result may include time information, such as occurrence time of and duration of the analysis result. Subsequently, the analysis result within a preset time range may be extracted based on the time information of the analysis result and compared with a preset rule.

Exemplarily, the processor 120 may analyze a single type of patient data to obtain an analysis result, or may analyze at least two types of patient data to obtain an analysis result. When analyzing at least two types of patient data, patient data of the same, similar or adjacent time may be selected for analysis based on time information carried in the patient data. Analyzing at least two types of patient data may include calculating a respective maximum, respective minimum, respective average, or a correlation of at least two types of patient data. For example, when calculating the correlation between a heart rate and a blood pressure, a suitable correlation calculation method, such as Pearson or Kendall, can be select, and start time and end time of heart rate data and of blood pressure can be set, wherein respective start time and end time of heart rate data and of blood pressure data can be exactly the same, or have a certain difference.

Exemplarily, the analysis result which is extracted from the patient data includes at least one of: a parameter value, an event, a result from secondary processing of parameter value, a result from secondary processing of event. The parameter value may include a parameter value which is extracted from monitoring data which is acquired by a sensor, such as a heart rate value which is extracted from heart rate data, a respiratory rate value which is extracted from respiratory data, a blood oxygen value which is extracted from blood oxygen data, etc. The parameter value may also include a measurement value which is extracted from imaging data, such as an ejection fraction (EF) which is extracted from an ultrasound image. The parameter value may also include a biochemical index value, which is extracted from biochemical test data, such as an arterial oxygen partial pressure (PaO2), a brain natriuretic peptide (BNP), etc. The parameter value may also include a height, weight, age of a patient, etc. The parameter value may also include a numerical code which is assigned to a non-quantitative value (such as mental state), for example, using a value of 1 to represent mental depression.

The result from a secondary processing of parameter value includes a calculation result which is obtained by using a mathematical method, such as, a variance, an average, a median, a maximum/minimum, a characteristic for trend of change, a measurement value of volatility, stationarity description, a random characteristic, a morphological pattern, a statistical parameter, etc. The above-mentioned characteristic for trend of change is a characteristic value which reflects a trend of change for a parameter value within a period of time, wherein the characteristic value can reflect a change direction or speed of the parameter value. For example, the heart rate increases monotonically at an average rate of +0.3 beats/minute within 1 hour, and the oxygenation index decreases monotonically at an average rate of -0.5 mmHg/minute within 1 hour, wherein the monotonic increasing can be represented by a value of 1, and the monotonic decreasing can be represented by a value of -1. The secondary processing of parameter value may also include further processing for at least two different processing results, such as obtaining a new parameter based on an average and a standard deviation of the heart rate.

The event, which is obtained based on the patient data, includes at least one of: an alarm for exceeding a threshold, an abnormal event, and a clinical event. Wherein, the alarm for exceeding a threshold is an alarm which is triggered, when the processor 120 monitors that a parameter value exceeds a preset alarm threshold, including but not limited to an alarm for a heart rate exceeding a threshold, an alarm for a blood pressure exceeding a threshold, an alarm for blood oxygen saturation exceeding a threshold, etc. Wherein "exceeding a preset alarm threshold" can be higher than a maximum alarm threshold or lower than a minimum alarm threshold. The abnormal event includes arrhythmia, and another abnormal event, which is obtained based on a waveform and another characteristic of the patient data and does not belong to the alarm for exceeding a threshold. The clinical event includes an examination event, a diagnosis event, a treatment event, a nursing event, etc., which is recorded in the patient data. When an event is obtained based on the patient data, time when the event occurs can also be recorded for subsequent analysis. The clinical event can reflect a patient state. For example, if the patient is receiving oxygen, it means that the patient may have unstable respiration and poor oxygenation, but the medical condition is mild and the patient can still breathe independently. If a ventilator is used to provide assisted respiration for the patient, the respiration instability of the patient is more serious. The patient may have lost consciousness and be unable to breathe independently. The ventilation method used by the ventilator can also reflect a severity of the patient state to a certain extent. For example, a patient using invasive ventilation may be more seriously ill than a patient using non-invasive ventilation. Administration of hypotensive drugs or antihypertensive drugs indicates that the patient may have instable circulatory system, so drugs are used to maintain stable blood pressure. Blood transfusion therapy to maintain adequate blood volume (including but not limited to supplementation with fresh plasma and red blood cells) indicates that the patient may have unstable circulation, and therefore blood transfusion is used to maintain a stable blood pressure. Fluid therapy to maintain fluid balance (including but not limited to crystalloid and colloid rehydration) indicates that the patient may have unstable circulation, so fluid rehydration is used to maintain body pressure balance.

The result from secondary processing of event includes information, such as an occurrence frequency of/occurrence time(s) of event, a trend of change for an occurrence frequency of/occurrence time(s) of event, a duration of event. The occurrence frequency of event includes, for example, a number of time(s) for a heart rate to exceed a threshold in four hours, a number of atrial fibrillation event(s) in two hours, a number of ventricular tachycardia event(s) in 30 minutes, etc. The trend of change for an occurrence frequency includes, for example, an increase or a decrease in the number of time(s) for a heart rate to exceed a threshold in four hours, compared with the number of time(s) for a heart rate to exceed a threshold in last four hours; an increase or a decrease in the number of atrial fibrillation event(s) in two hours, compared with the number of atrial fibrillation event(s) in last two hours. The duration includes, for example, respective durations of excessively high and low intracranial pressure (ICP), respective durations of excessively high and low end-tidal carbon dioxide (etCO2), a duration in which mean arterial pressure (MAP) <65 mmHg, etc.

Exemplarily, when secondary processing is performed on a parameter value or an event, a time range needs to be set so that the parameter data or event within the time range are secondary processed. For example, when calculating an average of a heart rate, in addition to setting a statistical calculation method used to calculate the average, it is also necessary to set the time range and extract heart rate values within the time range for calculating the average. The calculated average is an analysis result of the heart rate within the time range. That is, for each type of patient data, analysis results at different times can be obtained, and the analysis results at the same or similar time can be subsequently processed according to a corresponding relationship between the analysis results and time.

By integrating and extracting information contained in various types of patient data, a large number of analysis results are obtained. Afterwards, the system for processing vital information 100 can match and determine the analysis results according to a rule library, thereby determining the patient state according to the analysis results. The rule library contains a large number of preset rules. There is a pre-established corresponding relationship between preset rules and patient states. The preset rules can be formulated based on various methods, such as guideline rules, clinical consensus, clinical research, etc. Compared with determining the patient state based on the machine learning model, determining the patient state according to the preset rules is more accurate, the determined result is more controllable, and more in line with the clinical cognition of medical staff.

Specifically, the processor 120 determines one or more target rules which are satisfied by one or more analysis results, according to the one or more analysis results. Wherein each analysis result satisfies one target rule, or multiple analysis results satisfy one target rule, or each analysis result satisfies multiple target rules, depending on a preset corresponding relationship between the target rules and the analysis results. An analysis result satisfying a certain target rule, may be that the analysis result completely satisfies the target rule or that the analysis result is closest to the target rule.

Exemplarily, a target rule, which is related to a nervous system of the patient, includes an indicator which is related to a cranial nerve. A target rule, which is related to a circulatory system of the patient, includes an indicator which is related to hemodynamics or perfusion. A target rule, which is related to a respiratory system of the patient, includes an indicator which is related oxygenation. When determining a state of the nervous system of the patient, the processor 120 may select patient data which is related to the cranial nerve, extract an analysis result, and determine an indicator which is related to the cranial nerve and satisfied by the analysis result. When determining the state of the circulatory system of the patient, the processor 120 may select patient data which is related to hemodynamics or perfusion, extract an analysis result, and determine an indicator which is related to the hemodynamics or the perfusion and satisfied by the analysis result. When determining a state of the respiratory system of the patient, the processor 120 may select patient data which is related to oxygenation, extract an analysis result, and determine an indicator which is related to the oxygenation and satisfied by the analysis result.

Furthermore, the processor 120 may compare one or more analysis results with one or more preset rules in a rule library, thereby determining one or more target rules which are satisfied by the one or more analysis results among the one or more preset rules. One or more preset rules may be stored in the memory **110,** and the processor 120 calls the preset rule(s) from the memory 110. One or more preset rules may also be stored in a server, and the processor 120 calls the preset rule(s) from the server.

Each preset rule contains one or more preset conditions for one or more analysis results. Specifically, each preset rule may include a preset condition for single analysis result, or may include multiple preset conditions for multiple analysis results. The preset condition may include a threshold condition, a trend condition, a qualitative condition, etc. Each preset rule is defined with a corresponding analysis result, and the corresponding relationship between the preset rule and the analysis result can be selected to be included in the analysis result for comparison with the preset rule. For example, for a preset rule that "an average of a heart rate is greater than 90", the analysis result of "an average of a heart rate" is selected for comparison. Exemplarily, each preset rule also defines a duration. For example, for the preset rule that "an average of a heart rate is greater than 90 for more than 8 times in past 4 hours", a time range of "past 4 hours" and an analysis result of "an average of a heart rate is greater than 90" are selected to determine whether the average of a heart rate is greater than 90 for more than 8 times within the past 4 hours. If yes, the preset rule is satisfied, that is, the preset rule is a target rule. Otherwise, the preset rule is not satisfied, that is, the preset rule is not a target rule. For a preset rule "a number of occurrence time(s) for ventricular tachycardia exceeds 5 in past 30 minutes", a time range of "past 30 minutes" and an analysis result of "occurrence time(s) for ventricular tachycardia" are selected to determine whether a number of occurrence time(s) for ventricular tachycardia exceeds 5 in past 30 minutes. If yes, the preset rule is satisfied, otherwise, the preset rule is not satisfied.

When a same preset rule corresponds to at least two analysis results, the at least two analysis results may be extracted from the same type of patient data, for example, a same preset rule may include a preset condition for an average of a heart rate and a preset condition for a standard deviation of a heart rate. The at least two analysis results may also be extracted from different types of patient data, for example, a same preset rule may include a preset condition for an average of a heart rate and a preset condition for an average of blood oxygen.

At least two analysis results in the same preset rule may be a combination of a parameter value (or a result from secondary processing of parameter value) and an event (or a result from secondary processing of event). For example, at least two analysis results in a same preset rule can be a combination of a parameter value and an clinical event, thereby reflecting a change in the medical condition of the patient after providing a clinical treatment (such as ventilator-assisted respiration, medication, fluid infusion, blood supplementation, etc.), determining a treatment effect and a trend of development for the patient state. For example, a preset rule 1 is that after a treatment event of ventilator-assisted respiration occurs, SpO2 gradually increases, indicating that the state of the respiratory system has improved. A preset rule 2 is that after using analgesics for pain relief, etCO2 is too low for too long, or RR is too low for too long, or SpO2 is too low for too long, indicating that the analgesics may be excessive and suppress the respiratory system, and a dosage of the analgesics needs to be reduced. In some embodiments, the same preset rule may correspond to only one analysis result. For example, when a ventilator has been used to provide respiratory support for a patient, it can be considered that the patient is already in a very critical condition, so "using a ventilator" can be used as a preset rule alone, and the corresponding patient state is unable to breathe independently.

There is usually more than one type of patient data that changes due to a change in a patient state. The current system for processing vital information may monitor a trend of change for a single type of patient data, but does not take into account a correlation between trends of change for multiple types of patient data. An insignificant trend of change in a single type of patient data may not reflect the patient state alone, and may not attract the attention of medical staff, so that information contained therein cannot be effectively utilized. If multiple types of patient data simultaneously present a certain specific trend of change, it can more accurately reflect the patient state. Therefore, the system for processing vital information 100 of the embodiment of this disclosure comprehensively considers multiple trends of change to determine the patient state.

Specifically, at least one preset rule incorporates multiple trends of change for comprehensive consideration, and the multiple trends of change include at least a first trend of change for first information and a second trend of change for second information. The first information and the second information may be parameter values which are extracted from the patient data, results of secondary processing of parameter values, events which are extracted from patient data, or results of secondary processing of events. The first information and the second information may be extracted from the same or multiple types of patient data, or may be extracted from different types of patient data. The first trend of change and the second trend of change can be an uptrend, a downtrend, a fluctuating trend, an abrupt change, etc. The first trend of change and the second trend of change can also be a combination of multiple trends of change, such as first increasing and then decreasing, first decreasing and then fluctuating, etc.

For example, the first information is a heart rate value, the first trend of change is a trend of change for a heart rate value (hereinafter referred to as a trend of change for a heart rate), and the second information is a blood oxygen value, and the second trend of change is a trend of change for a blood oxygen value (hereinafter referred to as a trend of change for blood oxygen). After extracting the heart rate value from heart rate data, the trend of change for the heart rate value within a first time range is determined, for example, the heart rate value is determined to show an uptrend within the first time range. After extracting the blood oxygen value from blood oxygen data, the trend of change for the blood oxygen value within a second time range is determined, for example, the blood oxygen value is determined to show a downtrend within the second time range. The first time range and the second time range may be completely the same, at least partially the same, or close to each other. There are many ways to determine the trend of change. Taking the trend of change for the heart rate as an example, the heart rate values within the first time range can be linearly fitted, and the trend of change for the heart rate can be determined according to a slope of the fitted straight line; or the heart rate values within different time windows can be averaged, and the trend of change for the heart rate can be determined by comparing a size of the averages, etc. The embodiment of this disclosure does not limit a calculation method of the trend of change.

After obtaining the trend of change for the heart rate and the trend of change for the blood oxygen, the trend of change for the heart rate and the trend of change for the blood oxygen can be compared with a preset condition which is included in a preset rule to determine whether a combination of the trend of change for the heart rate and the trend of change for the blood oxygen can reflect a certain patient state. The preset condition used for comparison with the trend of change for the heart rate and the trend of change for the blood oxygen may be at least two preset conditions included in a same preset rule. The preset rule at least defines a preset trend of change for the heart rate and a preset trend of change for the blood oxygen, and comparing the trend of change for the heart rate and the trend of change for the blood oxygen at least includes determining whether an actual trend of change for the heart rate is consistent with the preset trend of change for the heart rate, and whether an actual trend of change for the blood oxygen is consistent with the preset trend of change for the blood oxygen. Data, such as physiological parameters of a patient rarely change individually, but mostly change in a coordinated manner. Analyzing and determining multiple trends of change at the same time is more in line with physiological laws and helps to improve the accuracy of identification for the patient state.

For example, in a preset rule which is associated with a deterioration of a circulatory state, a preset trend of change for the heart rate is an uptrend, and a preset trend of change for the blood oxygen is a downtrend. If the actual trend of change for the heart rate is an uptrend and the actual trend of change for the blood oxygen is a downtrend, then the preset rule is satisfied. If the actual trend of change for the heart rate and the actual trend of change for the blood oxygen are both uptrends or both downtrends, or the trend of change for the heart rate is a downtrend and the trend of change for the blood oxygen is an uptrend, then the preset rule is not satisfied. Other combinations of the trend of change for the heart rates and the trend of change for the blood oxygens may also represent other patient states. The preset rule may also define the trend of change for the heart rate and the trend of change for the blood oxygen as a trend of change in a same direction. If the trend of change for the heart rate and the trend of change for the blood oxygen are both uptrends or both downtrends, the preset rule is satisfied.

Furthermore, the preset condition also includes that the trend of change for the heart rate and the trend of change for the blood oxygen have a preset time correlation. Wherein the time correlation may include that occurrence time of the trend of change for the heart rate and occurrence time of the trend of change for the blood oxygen are at least partially the same, indicating that the heart rate and blood oxygen change synergistically. Optionally, the time correlation may include a time difference between occurrence time of the trend of change for the heart rate and occurrence time of the trend of change for the blood oxygen does not exceed a preset time difference, for example, the trend of change for the blood oxygen occurs within 5 minutes after the trend of change for the heart rate, indicating that changes in the heart rate and the blood oxygen have a certain correlation. Optionally, the time correlation may also include that the trend of change for the heart rate and the trend of change for the blood oxygen both occur within a same larger time range. Optionally, considering that changes in patient data may have a certain causal relationship, the preset condition may also define an order of the trend of change for the heart rate and the trend of change for the blood oxygen. For example, the trend of change for the blood oxygen must occur before the trend of change for the heart rate. Otherwise, even if the occurrence time of the both trends of change overlaps, the preset condition is not satisfied. Of course, the preset condition may not define the order of precedence.

After determining that one or more target rules are satisfied by the analysis result of the patient data, the processor 120 determines one or more patient states which correspond(s) to the target rule(s), and the patient state(s) is(are) a patient state which is determined by the system for processing vital information 100 for a currently monitored patient. The processor 120 may use the patient state(s) which correspond(s) to the target rule(s), as a determination result for the patient state according to a corresponding relationship between preset target rule(s) and preset patient state(s). The patient state determined by the processor 120 is usually an abnormal state because an abnormal state requires more attention from medical staff. However, the patient state may also be a normal state. For example, for the circulatory system, the processor 120 can determine that the patient state is an unstable circulatory state according to the target rule. When the unstable circulatory state is not determined, the circulatory system is defaulted to a stable circulatory state. The processor 120 can also determine that the patient state is a stable circulatory state according to some target rules.

The patient state in the embodiment of this disclosure may be a prediction result of the patient state in the future, or may be a determination result of the patient state at present. The patient state such as "instable circulatory system" mentioned below may refer to that the circulatory system of the patient is currently instable, or the circulatory system of the patient will be instable in the future. The prediction result of the patient state in the future represents a development trend of the patient state. Presenting the prediction result of the patient state in the future helps medical staff intervene early to avoid or slow down the deterioration of the patient state. Presenting the determination result of the patient state at present can also help medical staff to provide targeted treatment in a timely manner.

Previous monitoring device could only monitor a single physiological parameter, and inexperienced medical staff could not understand the reason for the alarm for a physiological parameter exceeding a threshold. Moreover, for critically ill patients, frequent alarms may cause alarm fatigue among medical staff, making them unable to pay attention to the deterioration of the patient state. In comparison, the system for processing vital information 100 of the embodiment of this disclosure can determine the patient state based on the patient data. By summarizing various types of patient data based on the system for processing vital information 100, there is no need for medical staff to conduct subjective analysis of large amounts of data, and the most clear, concise and most important information can be directly provided to medical staff. When the prediction result of the patient state in the future is outputted, treatment can be quickly activated, when the patient state shows signs of deterioration, preventing the disease from worsening as early as possible.

Exemplarily, the processor 120 may determine one or more patient states corresponding to one or more target rules according to corresponding relationship(s) between preset rule(s) and preset patient state(s). Corresponding relationship(s) between preset rule(s) and preset patient state(s) can be stored in the memory 110. After the processor 120 determines a target rule that matches the analysis result from the preset rule(s), the processor 120 determines one or more patient states which correspond(s) to the target rule, according to corresponding relationship(s) between preset rule(s) and preset patient state(s) which is(are) stored in the memory 110.

Wherein, each target rule corresponds to one patient state, that is, when one target rule is satisfied, the patient is determined to have a patient state which corresponds to the target rule. Optionally, each target rule corresponds to multiple patient states, that is, as long as one target rule is satisfied, the patient is determined to have multiple patient states at the same time. Optionally, multiple target rules correspond to one patient state, that is, only when multiple target rules are satisfied at the same time, the patient can be determined to have a patient state which corresponds to the multiple target rules. The patient state may include a state of multiple physiological structures of the patient. In some embodiments, the physiological structure includes at least a physiological system of the patient, wherein the physiological system includes at least one of: a nervous system, a circulatory system, and a respiratory system, which are the most concerned by clinical medical staff. In addition, the physiological system can also include a motor system, an endocrine system, a digestive system, a urinary system, a reproductive system, etc. The state of the physiological system is a direct reflection of an overall condition of the physiological system. Since the physiological system of human body is composed of multiple tissues or organs, previous monitoring device was unable to evaluate the overall condition of the physiological system. The system for processing vital information 100 of the embodiment of this disclosure integrates patient data of multiple types and sources, and realizes a comprehensive and general assessment of the state of the physiological system based on a rule library.

When the physiological system of the patient includes a nervous system, the target rule includes an indicator which is related to a cranial nerve of the patient; when the physiological system of the patient includes a circulatory system, the target rule includes an indicator which is related to hemodynamics or perfusion of the patient. When the physiological system of the patient includes a respiratory system, the target rule includes an indicator which is related to oxygenation of the patient.

In addition, the patient state may also include an overall state of the patient, a state of a physiological system, a state of an organ, a state of a physiological part, a state of a tissue, etc. The organ includes at least one of a brain, a heart, lungs, a liver, a stomach and kidneys. The physiological part includes head, chest, abdomen, etc.; the tissue includes muscle tissue, nervous tissue, epithelial tissue, etc.

The patient state may include one or more clinically defined macroscopic states of the patient as a whole, the physiological system, the organ, the physiological part, and the tissue, which state(s) reflect(s) an overall physiological function of that part, such as instable circulatory and insufficient perfusion of the circulatory system. The patient state(s) which correspond(s) to different physiological structures can be regarded as a summary of said physiological structure(s).

The patient state may specifically include a deterioration state of a physiological structure (such as a system, an organ, etc.), such as whether the physiological structure is currently deteriorating or whether the physiological structure is likely to deteriorate in the future. The patient state may also include whether an abnormality exists in the physiological structure (such as a system, an organ, etc.), a level of abnormality, a level of criticality, a level of nursing, etc. For example, the state of the circulatory system may include abnormal circulation or normal circulation, and the abnormal circulation may further include mild abnormal circulation or severe abnormal circulation. Optionally, the patient state may also include a specific disease which is related to the patient as a whole, the physiological system of the patient, the organ of the patient, the physiological part of the patient, or the tissue of the patient, such as acute respiratory distress syndrome (ARDS), respiratory failure, acute kidney injury (AKI), sepsis, heart failure, brain damage, etc. Exemplarily, the patient state may also include an unknown state or a suspected abnormal state. The patient state can be regarded as a comprehensive assessment result obtained by summarizing and analyzing the patient data by the system for processing vital information 100 instead of medical staff.

In some embodiments, the system for processing vital information 100 can determine the states of different physiological structures of the patient separately. For example, the system for processing vital information 100 can configure a corresponding type of patient data, a corresponding data analysis method, a corresponding preset rule for each physiological structure. After obtaining multiple types of patient data of the patient, the patient data can be classified according to a corresponding relationship between the physiological structure and the patient data, and then the patient data can be analyzed and determined according to the corresponding relationship between the physiological structure and the data analysis method and the preset rule. For example, based on the corresponding relationship between the respiratory system and the patient data, patient data which is related to the respiratory system can be extracted from multiple types of patient data, a relevant analysis result can be obtained according to a pre-configured data analysis method, and a target rule which is satisfied by the analysis result can be determined among multiple preset rules which are related to the respiratory system, so as to obtain a target rule which is related to the respiratory system. Finally, the state of the respiratory system can be obtained based on the corresponding relationship between the target rule and the patient state.

The system for processing vital information 100 may be pre-configured with multiple physiological structures, and a user may select a target physiological structure from the multiple physiological structures as required, thereby specifically viewing the patient state of a specific physiological structure. The system for processing vital information 100 can also configure different templates of combined sections for different types of patients or different types of users, and automatically select a target clinical section from multiple physiological structures according to a current type of patient or of user, thereby intelligently presenting the patient state which is concerned by different types of patients or different types of users. For example, for a patient with craniocerebral trauma, an elderly patient, a patient with heart failure, a patient requiring extracorporeal circulation support, and a patient with acute respiratory distress syndrome (ARDS), templates of combined sections, that satisfy the patient state of the patient which state needs to be paid attention to in the diagnosis and treatment of the patient, are defined. For cardiovascular doctors, respiratory therapists, and rehabilitation management personnel, templates of combined sections, which templates focus on the patient state which these clinical medical staff are concerned about, are defined separately. The system for processing vital information 100 may only determine the patient state corresponding to the target physiological structure, or the system for processing vital information 100 may determine the patient state corresponding to multiple physiological structures but only present the patient state corresponding to the target physiological structure.

In addition, the type of the patient data which corresponds to each physiological structure may also be customized by the user, and the type of the patient data which corresponds to each physiological structure may be determined according to a received user instruction. The user can select a target physiological structure and a type of the patient data which corresponds to the target physiological structure by considering factors, such as a medical condition of the patient (e.g., heart failure, injury, respiratory failure, etc.), hospital device, hospital condition, etc. At the same time, a template of combined sections can not only be used by the user who customized the template, but the user can also share or publish the customized template for others to use. In actual applications, the user can conveniently select a currently used template of combined sections from multiple templates of combined sections. The multiple templates of combined sections can be created by a current user or by other users.

In some embodiments, the processor 120 may also determine the patient state of the patient based on a first machine learning model for determining patient state. Wherein, the first machine learning model can be used as an auxiliary tool for preset rules to improve the accuracy of patient state.

Exemplarily, the processor 120 may input patient data or an analysis result which is extracted from the patient data, or a target rule which is satisfied by the analysis result into the first machine learning model, obtain a second patient state which is outputted by the first machine learning model, and obtain a final determination result of the patient state, based on the second patient state which is outputted by the first machine learning model and the first patient state which is determined based on the target rule. The final determination result of the patient state may include an union set of the first patient state and the second patient state, that is, as long as a patient state is determined based on any one of the rule library and the first machine learning model, the patient is considered to have this patient state. The final determination result of the patient state may also include an intersection set of the first patient state and the second patient state, that is, only when a patient state is determined based on both the rule library and the first machine learning model, the patient is considered to have said patient state. Optionally, the first patient state and the second patient state may be fused based on other strategies to obtain a more accurate patient state.

Alternatively, the processor 120 may also extract analysis result(s) from the patient data based on the first machine learning model, or perform secondary processing on the analysis result(s) extracted from the patient data based on the first machine learning model; the processor 120 may also perform secondary processing on the patient state obtained based on the target rule on the first machine learning model, etc.

Since training of the machine learning model is completely dependent on a training database, if an amount of data in the training database is small, or positive features are insufficient or unclear, an accuracy of the machine learning model will be reduced. In addition, the machine learning model has problems, such as unstable prediction results and unexplainable prediction results, making it difficult for medical staff to gain the trust of it. Therefore, the embodiment of this disclosure determines the patient state according to the preset rule, and the machine learning model can serve as an auxiliary to improve the accuracy of the patient state, and the reasons for the patient state can be explained by the target rule.

Optionally, the processor 120 may also independently obtain the patient state using a machine learning model. Specifically, the processor 120 can input multiple different types of patient data into the machine learning model to obtain the patient state which is outputted by the machine learning model, or the processor 120 can input analysis result(s) extracted from the patient data into the machine learning model to obtain the patient state which is outputted by the machine learning model. That is, the input of the machine learning model can be original patient data or the analysis result(s) extracted from the patient data. As artificial intelligence technology is researched and advanced, the accuracy of machine learning model in identifying patient state is also improving.

Conventional machine learning models cannot explain the reasons for the patient state. For this reason, the embodiment of this disclosure determines the target rule based on the analysis result extracted from the patient data, so that the reason for the patient state obtained by the machine learning model can be explained through the target rule. When the processor 120 controls the display 130 to simultaneously display the patient state and the target rule, the patient state may be determined based on the target rule, or based on the machine learning model, or determined in combination with the target rule and the machine learning model. Even if the patient state is not determined according to the target rule, the target rule is related to the patient state to some extent. In some embodiments, after determining the patient state according to the machine learning model, the target rule corresponding to the patient state can be reversed from the patient state, and the target rule can be displayed.

After determining the patient state based on any of the above methods, the processor 120 controls the display 130 to display indication information which indicates the patient state. Further, the processor 120 can also control the display 130 to display an explanatory description of the obtained patient state, thereby providing support for the patient state, increasing the credibility of the patient state, and better assisting user in diagnosing and treating the patient.

The explanatory description of the patient state may include at least a portion of one or more target rules. By presenting the indication information which indicates the patient state on the display 130, medical staff can be reminded to pay attention to the patient in a timely manner, especially to take timely response measures when the patient state is abnormal. Displaying the target rule which is related to the patient state can inform medical staff why the system for processing vital information 100 has derived the currently displayed patient state, thereby increasing the credibility of the patient state and, on the other hand, helping medical staff to provide timely symptomatic treatment. The processor 120 can update the patient state displayed on the display 130 according to a preset update cycle. The preset update cycle can be preset by the system for processing vital information 100, or can be input or modified by the user, and can be in minutes, hours, etc. The processor 120 can also continuously monitor the patient state in the background, and when it detects that the patient state has changed, it controls the display 130 to update the displayed patient state.

The indication information, which indicates the patient state, includes an overall assessment of the state of a physiological structure of the patient, such as a physiological system, a tissue, an organ, etc., which overall assessment briefly summarizes the overall state of the entire physiological structure and provides summary information about the entire physiological structure. The physiological structure includes a physiological system, a physiological organ, a physiological part, a tissue, a characteristic of physiological system or a characteristic of a physiological organ, of the patient. The physiological system includes at least one of: a motor system, a nervous system, an endocrine system, a circulatory system, a respiratory system, a digestive system, a urinary system and a reproductive system; the physiological organ includes at least one of: a brain, a heart, lungs, a liver, a stomach and kidneys; the physiological part includes at least one of: head, chest and abdomen; the tissue includes at least one of: a muscle tissue, a nervous tissue and an epithelial tissue; the characteristic of the physiological system or the characteristic of the physiological organ include at least one of: input and output amount, coagulation, nutrition, infection, blood sugar and a medical event.

Exemplarily, the processor 120 may control the display 130 to display indication information which indicates the patient state in a form of text or graph(s). Optionally, text and graph(s) can also be used in combination. When using a text mode, character strings related to the patient state can be pre-configured, including character strings representing an overall state, a physiological system, an organ, a physiological part or a tissue, of the patient, and character strings representing a specific state, such as circulatory system + possible shock/possible heart failure/possible internal bleeding, respiratory system + possible respiratory depression, nervous system + possible intracranial hemorrhage, urinary system + possible kidney failure, immune system + possible serious infection, etc. The character string may be in various forms as long as it can reflect the patient state. For example, a character string used to represent heart failure may be "Circulatory system may have heart failure", "Patient may have heart failure", "Patient is at risk of heart failure", etc. The character string can be pre-set by an expert for each patient state, or adjusted using natural language processing related methods to adapt to a current specific patient state. Exemplarily, the character string may also allow the user to configure or modify.

When a graph mode is used, a graph, which is related to each patient state that can vividly represent the patient state, can be pre-stored, and the graph can be called for display after the patient state is determined. The graph which indicates the patient state can correspond to an overall state, a physiological system, an organ, a physiological part or a tissue, of the patient, and the patient state can be presented by marking the graph with at least one of symbol information, color information and text information located on or near the graph. For example, displaying the graph representing the circulatory system in red indicates that the circulatory system is in an abnormal state, and displaying said graph in green indicates that the circulatory system is in a normal state. Graphs used to represent the patient state can be displayed separately in display areas which respectively corresponds to each patient state to facilitate medical staff to view them separately. For example, a graph used to represent the state of the circulatory system is displayed in a display area which corresponds to the circulatory system, a graph used to represent the state of the respiratory system is displayed in a display area which corresponds to the respiratory system, a graph used to represent the state of the nervous system is displayed in a display area which corresponds to the nervous system, and so on.

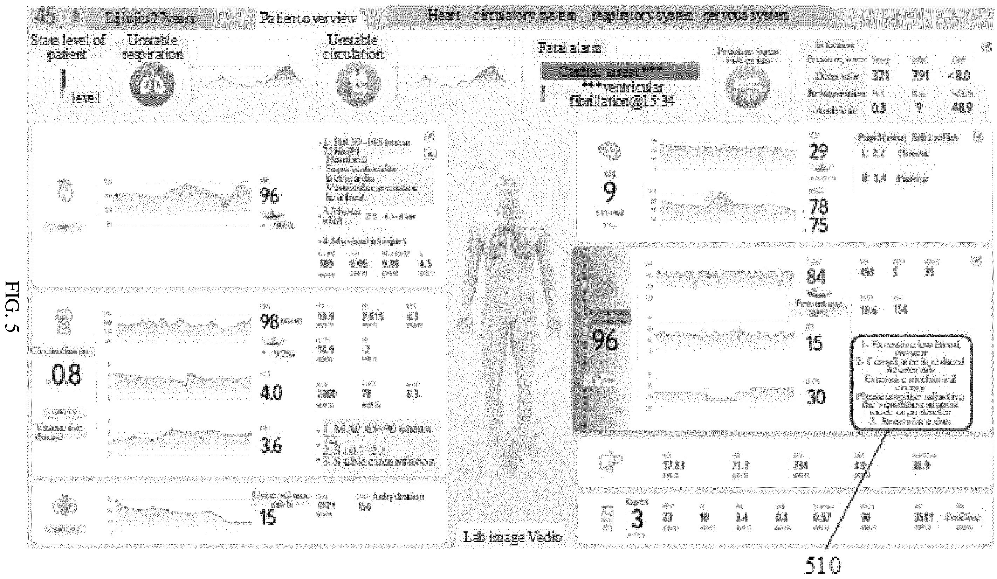

In some embodiments, as shown in FIG. 5, a graph which indicates the patient state may also be displayed in a same graph which indicates a body state, so as to facilitate medical staff to have an overview of the patient state. The graph which indicates a body state may be a full body diagram, a half body diagram, etc., in which multiple human organs or systems may be displayed. For example, a graph which indicates the state of the nervous system is displayed at a head position of the graph which indicates a body state, a graph which indicates the state of the circulatory system is displayed at a heart position of the graph which indicates a body state, a graph which indicates the state of the respiratory system is displayed at a chest position of the graph which indicates a body state, and a graph which indicates the state of the digestive system is displayed at an abdomen potion of the graph which indicates a body state, etc. The graph which indicates a body state may fully display multiple organs or systems, or may only display organ(s) or system(s) corresponding to target physiological structure(s), or may only display organ(s) or system(s) which is(are) currently in an abnormal state, or may only display organ(s) or system(s) which is(are) selected by the user. The graph which is used to represent the state of each system or of each organ may be a graph drawing of the corresponding system or organ, and a color or a dynamic change of the graph may be used to present the state of the corresponding system or organ. The overall state of the patient may also be represented by a human figure. For example, the overall state, such as sepsis or systemic infection, may be represented by a boundary line of the human figure.

Exemplarily, the processor 120 may control the display 130 to differentially display a prediction result of the future patient state from a determination result of the patient state at present, so that the user can determine which patient state is the current patient state and which patient state is a possible patient state in the future.

At least a portion of one or more target rules may be displayed adjacent to a corresponding patient state to demonstrate the correlation between the target rule(s) and the patient state. Displaying at least a portion of one or more target rules may include displaying several target rules with higher importance among multiple target rules. Since there may be a large number of target rules related to the patient state, selecting several target rules with higher importance for display helps medical staff to focus their attention without the need for manually screening the multiple rules. The displayed target rules can also be sorted according to a degree of importance, so that medical staff can give priority to target rules with higher importance. In some embodiments, the importance of the target rule may also be marked by color, graphs, text, etc.

Displaying at least a portion of one or more target rules may also include displaying a number of preset conditions with higher importance among the multiple target rules. As described above, each target rule may include one or more preset conditions, and preset condition(s) which is(are) finally displayed may be a portion of the conditions in each target rule, that is, the preset condition(s) that medical staff are more concerned about may be selected in each rule for display without displaying other preset condition(s) in said rule. For example, some target rules include preset conditions for age, weight, medical condition, etc., of the patient. These preset conditions are related to thresholds for the preset conditions of vital sign data. Although they have an important impact on the determination of the patient state, medical staff usually do not pay attention to this type of information. Therefore, when displaying the target rules, only the preset conditions for vital sign parameters can be displayed, without displaying the preset conditions for information such as age and weight. Of course, there may be many selection criteria for the preset conditions that are finally displayed, which can be set specifically according to clinical requirements, and the embodiments of this disclosure do not limit this.

In some embodiments, the processor 120 may further determine a state level which represents a severity of the patient state, and control the display 130 to display indication information which indicates the state level. The processor 120 may combine multiple patient states to jointly determine a comprehensive state level, for example, the overall state of the patient is a level which represents severe deterioration. The comprehensive state level may be used to remind medical staff which patient needs the most attention. Alternatively, the processor 120 may determine a separate state level for each patient state, such as separately determining respective state level of the respiratory system, nervous system, and circulatory system; the separate state level may be used to indicate the medical staff which aspect of the patient currently requires the most attention.

When a patient has some abnormal patient states, displaying the target rule can indicate the reason for the patient state, but it is still difficult for inexperienced medical staff to determine the root reason for the patient state. Therefore, the embodiment of this disclosure also determines a physiological reason which results in the patient state, and controls the display 130 to display indication information which indicates the physiological reason, thereby providing the user with a deeper explanatory description of the patient state. Wherein, the physiological reason is a physiological reason for a current patient state, which reason is determined based on biomedical principles, clinical experience, etc., and can specifically be a certain disease or injury (i.e., a reason for the disease). The physiological reason may also be the patient state on the other hand. For example, a physiological reason which is related to the patient state of the physiological system may be a state of a certain tissue or organ in the physiological system, for example, deterioration of the state of a certain tissue or organ leads to deterioration of the entire system; or, the physiological reason may be a state of another physiological system, for example, deterioration of the state of one physiological system leads to deterioration of the state of another physiological system. In summary, there is a causal relationship between the physiological reason and the patient state.

Exemplarily, the processor 120 may determine the physiological reason which results in the patient state based on corresponding relationship(s) between preset rule(s) and preset physiological reason(s). The memory 110 can store the corresponding relationship(s) between preset rule(s) and preset physiological reason(s). After determining that the analysis result, which is extracted from the patient data, satisfies at least one target rule, the physiological reason for the patient state corresponding to the at least one target rule is determined based on the corresponding relationship. Each preset rule may correspond to one physiological reason, or each preset rule may correspond to multiple physiological reasons, or multiple preset rules may correspond to one physiological reason.

In some embodiments, the processor 120 may also determine the physiological reason for the patient state based on a second machine learning model for determining physiological reason. Specifically, the patient data, one or more analysis results which are extracted from the patient data, or the target rules which are satisfied by the analysis results, may be inputted into the second machine learning model, so as to obtain the physiological reason which is outputted by the second machine learning model.

In order to reflect a correlation between the physiological reason and the target rule, the processor 120 may control the display 130 to associatively display at least a portion of the target rule and the indication information which indicates the physiological reason. The associated displaying may include presenting a correlation between the physiological reason and the target rule through specific character(s), or displaying the associated target rule and the physiological reason adjacent to each other or in the same area.

For example, referring to FIG. 2, based on the patient data and multiple preset rules, the processor 120 determines that the target rules, which are satisfied by the analysis results which are obtained from the patient data, include: target rule 1, MAP is between 52-94 mmHg, and is less than 65 mmHg in 22% time; target rule 2, S1>0.7, and MS1>0.9, and AS1>47; Target rule 3, HR increases, and SpO2 decreases.

Based on the corresponding relationship between the above target rule and the patient state, the processor 120 determines that the state of the circulatory system of the patient is "instable circulatory system". Based on the corresponding relationship between the above target rule and the physiological reason, the processor 120 determines that the physiological reason which corresponds to target rule 1 is that a risk of long-term insufficient perfusion exists; the physiological reason which corresponds to target rule 2 is possible insufficient perfusion; and the physiological reason which corresponds to target rule 3 is insufficient oxygenation. Based on the above information, the processor 120 controls the display 130 to display the indication information which indicates a physiological reason for instable circulatory system, to display the target rule 1, the target rule 2 and the target rule 3, and to display the physiological reason which corresponds to each target rule after the target rule.

In the above description, the processor 120 controls the display to display the target rule to indicate the reason for the patient state. In addition, the processor 120 can also control the display 130 to display at least a portion of information which is extracted from the patient data and used to determine the patient state, so as to indicate medical staff on the basis for obtaining the patient state. For example, when the patient state is determined based on the first machine learning model, key information, which is extracted from the patient data and used by the machine learning model to obtain the patient state, can be determined and displayed. For example, the first machine learning model can output the key information that has greatest contribution rate to the patient state while outputting the patient state, and displaying the key information which explains why the patient state is obtained by the first machine learning model, thereby increasing the credibility of the patient state. Optionally, the machine learning model can also output a weight of contribution which is made by each key information, and the display 130 can display each key information in order from a high weight to a low weight.

For example, when the physiological system of the patient includes the nervous system of the patient, the information which is extracted from the patient data includes an indicator which is related to a cranial nerve; when the physiological system of the patient includes the circulatory system of the patient, the information which is extracted from the patient data includes an indicator which is related to hemodynamics or perfusion; when the physiological system of the patient includes the respiratory system of the patient, the information which is extracted from the patient data includes an indicator which is related to oxygenation.

Exemplarily, the information, which is extracted from the patient data, may include a parameter value which is extracted from the patient data, a result from secondary processing of parameter value, an event, and a result from secondary processing of event, as described above. In addition, the information, which is extracted from the patient data, may also be used to obtain a certain waveform graph or a trend graph based on the patient state, and its presentation form may be directly presenting the waveform graph, the trend graph, other statistical graph(s), etc., and there is no need to present the information which is extracted from the patient data in a form of character(s).

The waveform graph reflects changes of patient data over time in one or more cycles. For example, changes in body surface potential are recorded in a time sequence of cardiac excitement to form a continuous curve, which is an ECG waveform graph; a corresponding set of waveforms can appear on the ECG waveform graph for each cardiac cycle. Similar waveforms include a waveform of blood oxygen saturation, a waveform of end-tidal carbon dioxide, etc. The trend graph is configured to reflect a trend of one or more types of patient data over time, and has a longer time dimension than the waveform graph. A vertical axis of the trend graph can be either absolute values which are acquired at a certain sampling rate, or averages which are acquired and calculated at a certain sampling rate in each fixed time period. The trend graph may be one of or any combination of: a curve chart, a histogram, a bar chart, a box plot, a scatter plot, and a line chart.

In some embodiments, a trend graph may be used to reflect a trend of change over time in an event which is identified from the patient data, such as an occurrence pattern, a development situation, an occurrence frequency, and a trend, etc., of the event, which changes and develops over time. Wherein, "occurrence frequency" can be a frequency of occurrence of an event within a minimum unit time length, while the occurrence pattern, development situation, trend, etc., are not limited to the occurrence frequency, but can be marked according to an actual situation of the event. For example, taking an atrial fibrillation event as an example, each time an atrial fibrillation event occurs, a specific graph/symbol is used for mark. When the atrial fibrillation event occurs continuously for a long time, a length of a horizontal axis, which length is spanned by a specific graph/symbol, can be used to reflect a duration of the atrial fibrillation event. A same trend graph can also be used to present changes of at least two types of patient data over time. For example, at least two of: a trend graph of a heart rate, a trend graph of a pulse rate, a trend graph of blood oxygen, a trend graph of non-invasive blood pressure, a trend graph of invasive blood pressure, a trend graph of respiration, a trend graph of body temperature, a trend graph of cardiac output, electrocardiogram ST segment, electrocardiogram QT interval, blood sugar, brain oxygen, and urine volume, can be displayed simultaneously, that is, sharing a same time axis, or aligning time axes for display. Displaying trend graphs of at least two types of the patient data simultaneously helps to jointly present a correlation of changes in different types of patient data during the monitoring period. In some embodiments, the processor 120 may also perform a noise assessment on the patient data to confirm a data quality of the patient data. The noise assessment may help determine whether the currently obtained patient state is reliable. For example, when the data quality of a certain type of patient data is too poor, the patient state is not determined based on said patient data to prevent an output of unreliable information which information interferes normal clinical diagnosis and treatment of the doctor. Alternatively, a parameter of data quality may be presented while displaying the patient state. The parameter of data quality reflects the data quality of the patient data used to determine the patient state, so as to assist medical staff in confirming the credibility of the currently displayed patient state.

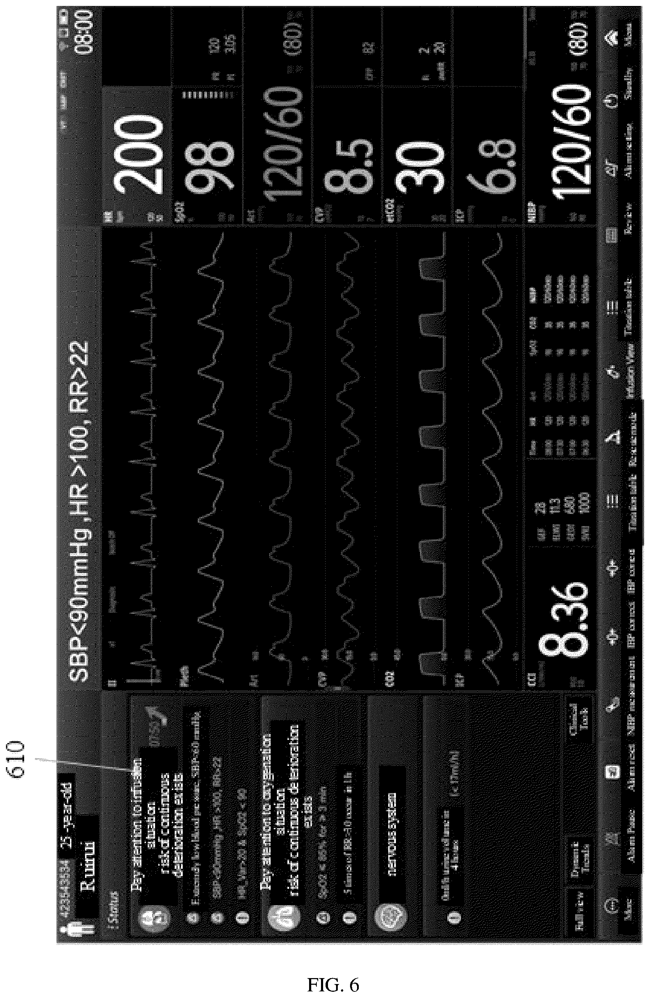

As shown in FIG. 6, the above-mentioned patient state, target rule, physiological reason and other information, according to the embodiment of this disclosure can be displayed in a patient state window 610. The patient state window 610 can be a window which is superimposed on a conventional monitoring interface. The user can choose whether to display the patient state window, or the patient state window pops up automatically when an abnormality occurs in the patient state. Alternatively, the above-mentioned patient state, target rule, physiological reason and other information can be displayed in a patient state area of a conventional monitoring interface, that is, a fixed display area is allocated to the patient state, target rule, physiological reason and other information in the conventional monitoring interface. The conventional monitoring interface displays real-time monitoring values, waveform graphs, parameter alarm information, etc. of conventional vital sign data.

Optionally, as shown in FIG. 5, the above-mentioned patient state, target rule, physiological reason and other information can also be displayed in an interface for monitoring the patient state. The interface for monitoring the patient state displays information for multiple physiological structures of the patient, and the information for at least one physiological structure includes summary information 510 which corresponds to said physiological structure. The user can switch between the conventional monitoring interface and the interface for monitoring the patient state. For example, the user can enter the interface for monitoring the patient state of FIG. 5 by clicking a control on the conventional monitoring interface of FIG. 6; or the interface for monitoring the patient state can be directly presented on the display. In some embodiments, the above information is provided to the doctor by outputting an electronic report or printing a paper report through a printing device.

For example, as shown in FIG. 5, in the interface for monitoring the patient state, information of different physiological structures can be displayed in different display areas. For example, each physiological structure corresponds to one independent card, or information of different physiological structures can be distinguished by different colors or boundary lines. Summary information for at least one physiological structure is displayed in a display area which corresponds to said physiological structure.

Exemplarily, the information, which is extracted from the patient data and displayed in a display area, which area corresponds to the respiratory system, includes at least one of: oxygenation index (PaO2/FiO2), blood oxygen saturation (SpO2), respiratory rate (RR), inspired oxygen concentration (FiO2), end-tidal carbon dioxide, a parameter of blood gas analysis, and a parameter of a ventilator or of an oxygen therapy device, etc. Wherein, a measured value of the parameter of blood gas analysis includes lactate (Lac), arterial oxygen partial pressure (PaO2), and arterial carbon dioxide partial pressure (PaCO2), etc. A measured value of the parameter of a ventilator includes tidal volume (Tv), positive end-expiratory pressure (PEEP), and a current oxygen supply mode of patient, for example, using SIMV ventilation mode, intubation or mask to supply oxygen, etc. The display interface may display graph(s) for trends of change for the measured values of the parameter(s) and/or for the parameter(s). A measured value that exceeds a normal range may be marked for highlighting. Exemplarily, a change between a currently measured parameter and a last measured parameter may also be provided. For a parameter only a measure value of which is displayed, a numerical value and a graph for trend of change within a preset time period can also be displayed, in response to a selection instruction for the measure value of said parameter

A display area which corresponds to the respiratory system also displays summary information 510 of the respiratory system. Summary information of the respiratory system may include a general summary of the overall state of the respiratory system (such as unstable respiration), a current problem which the respiratory system has (such as transient hypoxemia, intermittent decrease in compliance), a suggestion for a user (such as please consider adjusting ventilation support mode or parameter), a possible risk (a risk of pressure injury), etc. The information, which is extracted from data, which data is related to the respiratory system, and is displayed on the display interface, may be the same as or different from the information which is used to obtain the state of the respiratory system. The target rule for obtaining the state of the respiratory system mainly includes an indicator which is related to oxygenation of the patient.

Exemplarily, an entrance for a manual tool for clinical assessment, which tool is related to the respiratory system, is also displayed in the display area which corresponds to the respiratory system, and the user can select the entrance to enable the manual tool for clinical assessment.

The information, which is displayed in a display area which corresponds to the circulatory system, mainly includes information which is extracted from data which is related to hemodynamics and perfusion. The patient data which is related to the circulatory system include shock index, blood pressure, cardiac output, lactate (Lac), as well as a laboratory indicator and a hemodynamic parameter which are related to hemodynamics and perfusion. Wherein, the blood pressure can be an invasive blood pressure or a non-invasive blood pressure. The laboratory indicator includes but is not limited to hemoglobin (Hb or HGB), red blood cell count (RBC), pH, HCO3, and base excess (BE). The hemodynamic parameter includes but is not limited to a central venous pressure (CVP), a systemic vascular resistance index (SVRI), an extra vascular lung water index (ELWI), and a central venous oxygen saturation (ScvO2). In addition, the patient data which is related to the circulatory system also includes information for a supporting device or a treatment device, which is related to the circulatory system, specifically including a treatment mode which is used by the supporting device or treatment device, a key parameter of device, etc., such as whether to use an extracorporeal circulation support device, such as ECMO, whether to use intra-aortic ballon pump (IABP); whether to use vasoactive drug, etc.

A display area, which corresponds to the circulatory system, also displays the state of the circulatory system, such as unstable circulation, insufficient perfusion, etc. The information, which is extracted from the data related to the circulatory system, and displayed on the display interface, may be the same as or different from the information which is used to obtain the state of the circulatory system. The target rule for obtaining the state of the circulatory system mainly includes an indicator which relates to hemodynamic or perfusion.

The information, which is displayed in a display area which corresponds to the nervous system, mainly includes information which is extracted from the patient data which is related to a cranial nerve. Exemplarily, the patient data which is related to the nervous system includes a consciousness score, indexes for cerebral blood pressure and blood oxygen, a clinical assessment result which is related to the nervous system, etc. The commonly used consciousness score in clinical practice is a Glasgow coma scale (GCS) score, but the users are also allowed to define their own consciousness scoring rules. A clinical assessment result, which is related to the nervous system, includes an assessment result for a pupil size, an assessment result for pupil light reflex, an assessment result for strength of limb muscle, etc.

A display area, which corresponds to the nervous system, also displays the state of the nervous system. The information, which is extracted from the data related to the nervous system, and displayed on the display interface, may be the same as or different from the information which is used to obtain the state of the nervous system. The target rule for obtaining the state of the nervous system mainly includes an indicator which is related to the cranial nerve of the patient.

The information, which is displayed in a display area which corresponds to the heart, mainly includes an assessment result which is related to a cardiac risk, such as thrombolysis and thrombin inhibition in myocardial Infarction (TIMI) score. If the subject has undergone a GRACE (Global Registry of Acute Coronary Event) assessment, the assessment result, which is related to a cardiac risk, may also include a GRACE score. **In** addition, the information, which is displayed in a display area which corresponds to the heart, also includes an biochemical indicator which is related to a heart rate and heart, such as creatine kinase isoenzyme (CK-MB), troponin (cTn), natriuretic peptide (NT-proBNP), etc.; as well as an alarm event which is related to heart, such as an event of ST segment elevation or an event of ST segment depression. For a serious arrhythmia event, event information for fatal arrhythmia can be displayed, including a number of times arrhythmia occurs in a past time period.

The information, which is displayed in a display area which corresponds to the liver, mainly includes an assessment indicator for a liver function, such as alanine aminotransferase (ALT), γ-glutamyl transaminase (GGT), total bilirubin (Tbil), direct bilirubin (Dbil), and blood ammonia (AMM). For indicators with multiple assessments, change information between a latest indicator and a previous indicator is presented. User can customize the assessment indicator for the liver function as they wish to view. For example, if the medical subject undergoes an assessment for a liver function, such as obtaining a Child-Pugh grading score, information for the score result is provided in the display area which corresponds to a liver section.

The information, which is displayed in a display area which corresponds to kidneys, mainly includes an urine volume, a liquid intake and output volume. The liquid intake and output volume includes a liquid intake volume and a liquid output volume, wherein the liquid intake volume includes a total intake volume in 24 hours, and a liquid intake volume which is pumped into the human body by an infusion pump within 24 hours. Furthermore, the liquid intake volume may also include a liquid intake volume from dietary, and the likes. The liquid output volume includes an urine volume within 24 hours, a drainage fluid volume within 24 hours, a dehydration fluid volume from other device(s), etc. The liquid output volume can also include a sweating volume, an excretion volume, a vomiting volume, a bleeding volume, etc.

Exemplarily, continuing to refer to FIG. 5, a graph which indicates a body state is also displayed in the interface for monitoring the patient state, and the graph which indicates a body state can provide an overview of the patient state. The graph which indicates a body state may be displayed at a center of the interface for monitoring the patient state, but is not limited thereto. The graph which indicates a body state may fully display multiple organs or systems, or may only display organ(s) or system(s) which correspond(s) to target physiological structure(s), organ(s) or system(s) currently in an abnormal state, or organ(s) or system(s) selected by the user. The graph which indicates a state of each system or organ may be a diagram drawing a corresponding system or organ, and color or a dynamic change of the diagram may be used to present the state of the corresponding system or organ. The overall state of the patient may also be represented by a human figure. For example, the overall state such as sepsis or systemic infection may be represented by the boundary line of the human figure.

**In** summary, the system for processing vital information 100 of the embodiment of this disclosure determines a patient state based on a target rule thus improving an accuracy of the patient state, displays the patient state along with a target rule which is used to explain the patient state, thus enhancing a credibility of the patient state, and assisting medical staff to obtain a timely and targeted treatment for the patient.

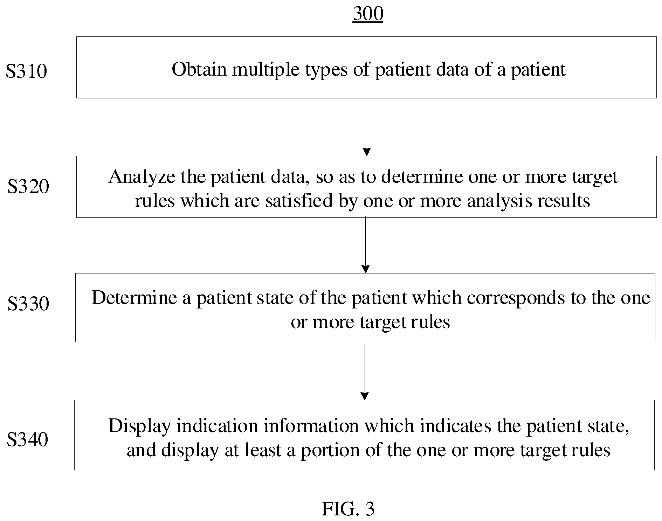

**In** the followings, a method for processing vital information according to an embodiment of this disclosure is described with reference to FIG. 3. FIG. 3 is a flow chart of a method for processing vital information according to an embodiment of this disclosure.

As shown in FIG. 3, the method for processing vital information 300 of the embodiment of this disclosure includes the following steps.

**In** step S310, obtain multiple types of patient data of a patient.

**In** step S320, analyze the patient data, so as to determine one or more target rules which are satisfied by one or more analysis results.

**In** step S330, determine a patient state of the patient which corresponds to the one or more target rules.

**In** step S340, display indication information which indicates the patient state, and display at least a portion of the one or more target rules.

Illustratively, the patient state at least includes a state of a physiological system of the patient; the physiological system includes at least one of a nervous system, a circulatory system, and a respiratory system, of the patient. When the physiological system of the patient includes the nervous system of the patient, the one or more target rules include an indicator which is related to a cranial nerve; when the physiological system of the patient includes the circulatory system of the patient, the one or more target rules include an indicator which is related to hemodynamics or perfusion; when the physiological system of the patient includes the respiratory system of the patient, the one or more target rules include an indicator which is related to oxygenation.

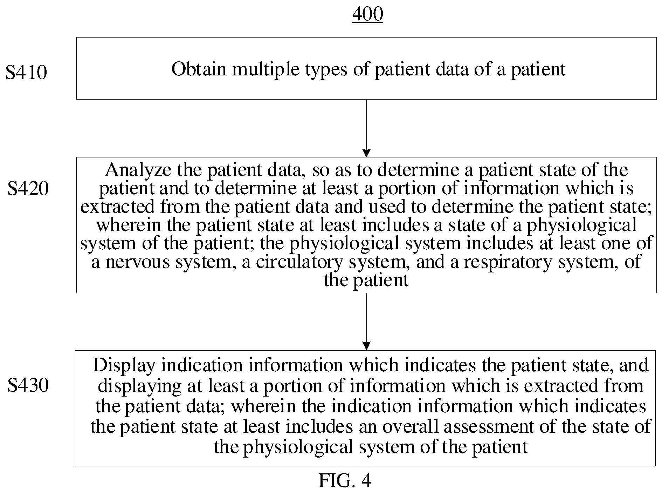

As shown in FIG. 4, the method for processing vital information 400 of the embodiment of this disclosure includes following steps.

In step S410, obtain multiple types of patient data of a patient.

In step S420, analyze the patient data, so as to determine a patient state of the patient and to determine at least a portion of information which is extracted from the patient data and used to determine the patient state; wherein the patient state at least includes a state of a physiological system of the patient; the physiological system includes at least one of a nervous system, a circulatory system, and a respiratory system, of the patient.

In step S430, display indication information which indicates the patient state, and displaying at least a portion of the information which is extracted from the patient data; wherein the indication information which indicates the patient state at least includes an overall assessment of the state of the physiological system of the patient.

Illustratively, when the physiological system of the patient includes the nervous system of the patient, the information which is extracted from the patient data includes an indicator which is related to a cranial nerve; when the physiological system of the patient includes the circulatory system of the patient, the information which is extracted from the patient data includes an indicator which is related to hemodynamics or perfusion; when the physiological system of the patient includes the respiratory system of the patient, the information which is extracted from the patient data includes an indicator which is related to oxygenation.

The method for processing vital information 300 according to an embodiment of this disclosure and the method for processing vital information 400 according to an embodiment of this disclosure can be implemented in the system for processing vital information 100 described above. Specifically, each step of the method for processing vital information can be executed by the processor 120 of the system for processing vital information 100. The specific details of the method for processing vital information can be made with reference to the above description of the system for processing vital information 100, which are not repeated here.

Although the exemplary embodiments are described here with reference to the accompanying drawings, it should be understood that the aforementioned exemplary embodiments are only illustrative and are not intended to limit the scope of this disclosure. Ordinary technical personnel in this field can make various changes and modifications without deviating from the scope and spirit of this disclosure. All these changes and modifications are intended to be included within the scope of this disclosure as claimed in the attached claims.

Ordinary technical personnel in this field can realize that the units and algorithm steps described in combination with this disclosure can be implemented in electronic hardware, or a combination of computer software and electronic hardware. Whether these functions are executed in hardware or software depends on the specific application and design constraints of the technical solution. Professional technicians may use different methods to implement the described functions for each specific application, but such implementation should not be considered beyond the scope of this disclosure.

**In** the several embodiments provided in this disclosure, it should be understood that the disclosed devices and methods can be implemented in other ways. For example, the device embodiments described above are only schematic. For example, the division of the units is only a logical functional division, and there may be other division methods in actual implementations. For example, multiple units or components can be combined or integrated into another device, or some characteristics can be ignored or not executed.

The disclosure provided here provides a large number of specific details. However, it can be understood that the embodiments of this disclosure can be practiced without these specific details. **In** some examples, well-known methods, structures, and techniques are not shown in detail to avoid blurring the understanding of this disclosure.

Similarly, it should be understood that in order to streamline this disclosure and assist in understanding one or more of the various aspects of this disclosure, in the description of exemplary embodiments of this disclosure, the various characteristics of this disclosure are sometimes grouped together into a single embodiment, diagram, or description thereof. However, the method of this disclosure should not be interpreted as reflecting the intention that the claimed protection of this disclosure requires more characteristics than those explicitly recorded in each claim. More precisely, as reflected in the corresponding claims, the inventive point is that the corresponding technical problem can be solved with less than all characteristics of a single disclosed embodiment. Therefore, the claims following the specific implementation method are explicitly incorporated into the specific implementation method, where each claim itself serves as a separate embodiment of this disclosure.

Ordinary technical personnel in this field can understand that, in addition to mutual exclusion between characteristics, any combination can be configured to combine all characteristics disclosed in this disclosure (including accompanying claims, abstract, and drawings), as well as all processes or units of any method or device so disclosed. Unless otherwise explicitly stated, each characteristic disclosed in this disclosure (including accompanying claims, abstract, and accompanying drawings) may be replaced by alternative characteristics that provide the same, equivalent, or similar purpose.

**In** addition, Ordinary technical personnel in this field can understand that although some embodiments described herein include certain characteristics rather than other characteristics included in other embodiments, the combination of characteristics of different embodiments means that they are within the scope of this disclosure and form different embodiments. For example, in the claims, any one of the claimed embodiments can be used in any combination.

The various component embodiments of this disclosure can be implemented in hardware, software modules running on one or more processors, or a combination thereof. Ordinary technical personnel in this field should understand that a microprocessor or digital signal processor (DSP) can be used in practice to implement some or all of the functions of some modules according to the embodiments of this disclosure. This disclosure can also be implemented as a partial or complete device program (such as a computer program and a computer program product) for executing the method described herein. The program implementing this disclosure can be stored at a computer-readable medium or can have the form of one or more signals. Such signals can be downloaded from internet websites, provided on carrier signals, or in any other form.

It should be noted that the above embodiments illustrate this disclosure rather than limit it, and Ordinary technical personnel in this field can design alternative embodiments without departing from the scope of the attached claims. **In** the claims, any reference symbol between parentheses should not be constructed as a restriction on the claims. This disclosure can be implemented with the help of hardware consisting of several different components and with the help of appropriately programmed computers. Among the unit claims that list several devices, several of these devices can be specifically embodied through the same hardware item. The use of words first, second, and third does not indicate any order. These words can be interpreted as names.

The above is only the specific implementation method or explanation of the specific implementation method of this disclosure. The protection scope of this disclosure is not limited to this. Any technical personnel familiar with the technical field can easily think of changes or replacements within the technical scope disclosed in this disclosure, and those changes or replacements should fall into the protection scope of this disclosure. The protection scope of this disclosure shall be based on the protection scope of the claims.

## Claims

1. A system for processing vital information, **characterized in that**, comprising a memory, a processor, and a display; wherein the memory is configured to store an executable program, and the processor is configured to execute the executable program, so as to enable the processor to perform following operations:
obtaining patient data of a patient;
analyzing the patient data, so as to determine one or more target rules which are satisfied by the patient data;
determining a patient state of the patient which corresponds to the one or more target rules, wherein the patient state at least comprises a state of a physiological system of the patient; the physiological system of the patient comprises at least one of: a nervous system, a circulatory system, and a respiratory system, of the patient; and
controlling the display to display indication information which indicates the patient state, and to display at least a portion of the one or more target rules; wherein the indication information which indicates the patient state at least comprises an overall assessment of the state of the physiological system of the patient; wherein
when the physiological system of the patient comprises the nervous system of the patient, the one or more target rules comprise an indicator which is related to a cranial nerve;
when the physiological system of the patient comprises the circulatory system of the patient, the one or more target rules comprise an indicator which is related to hemodynamics or perfusion;
when the physiological system of the patient comprises the respiratory system of the patient, the one or more target rules comprise an indicator which is related to oxygenation.

2. The system for processing vital information according to claim 1, **characterized in that**, the memory is further configured to store one or more preset rules;
analyzing the patient data, so as to determine one or more target rules which are satisfied by the patient data, comprises:
analyzing the patient data, so as to obtain one or more analysis results;
determining whether the one or more analysis results satisfy the one or more preset rules, and using preset rule(s) which is(are) satisfied as the one or more target rules.

3. The system for processing vital information according to claim 2, **characterized in that**, the memory is further configured to store corresponding relationship(s) between the preset rule(s) and preset patient state(s);
determining a patient state of the patient which corresponds to the one or more target rules, comprises:
determining the patient state of the patient which corresponds to the one or more target rules, according to said corresponding relationship(s).

4. The system for processing vital information according to claim 2 or claim 3, **characterized in that**, each preset rule comprises a preset condition for single analysis result, and/or, each preset rule comprises preset condition(s) for at least two analysis results.

5. The system for processing vital information according to any one of claims 2-4, **characterized in that**, analyzing the patient data, so as to obtain one or more analysis results, comprises:
analyzing a single type of the patient data, so as to obtain the analysis result(s); and/or
analyzing at least two types of the patient data, so as to obtain the analysis result(s).

6. The system for processing vital information according to any one of claims 2-5, **characterized in that**, the analysis result comprises at least one of: a parameter value, an event, a result from secondary processing of parameter value, a result from secondary processing of event.

7. The system for processing vital information according to claim 6, **characterized in that**, the event comprises at least one of: an alarm for exceeding a threshold, an abnormal event, and a clinical event.

8. The system for processing vital information according to claim 1, **characterized in that**, determining a patient state of the patient which corresponds to the one or more target rules, comprises:
using the patient data and a first machine learning model for determining patient state to determine the patient state of the patient which corresponds to the one or more target rules.

9. The system for processing vital information according to claim 1, **characterized in that**, the processor is further configured to perform following operations:
determining a state level which represents a severity of the patient state, and
controlling the display to display indication information which indicates the state level.

10. The system for processing vital information according to claim **1, characterized in that**, the processor is further configured to perform following operations:
determining a physiological reason which results in the patient state according to the patient data; and
controlling the display to display indication information which indicates the physiological reason.

11. The system for processing vital information according to claim 10, **characterized in that**, the memory is further configured to store one or more preset rules, and corresponding relationship(s) between the preset rule(s) and preset physiological reason(s);
determining a physiological reason which results in the patient state according to the patient data, comprises:
analyzing the patient data, so as to obtain one or more analysis results;
determining whether the one or more analysis results satisfy the one or more preset rules, and using preset rule(s) which is(are) satisfied as the one or more target rules; and
determining the physiological reason which results in the patient state according to said corresponding relationship(s).

12. The system for processing vital information according to claim 11, **characterized in that**, the processor is further configured to perform a following operation:
controlling the display to associatively display at least a portion of the one or more target rules, and the physiological reason.

13. The system for processing vital information according to claim 10, **characterized in that**, determining a physiological reason which results in the patient state according to the patient data, comprises:
using the patient data and a second machine learning model for determining physiological reason to determine the physiological reason which results in the patient state.

14. The system for processing vital information according to any one of claims 1-13, **characterized in that**, the physiological system of the patient further comprises at least one of: a motor system, an endocrine system, a digestive system, a urinary system and a reproductive system, of the patient.

15. The system for processing vital information according to any one of claims 1-14, **characterized in that**, the patient state of the patient further comprises at least one of: an overall state of the patient, a state of an organ of the patient, a state of a physiological part of the patient, a state of a tissue of the patient.

16. The system for processing vital information according to claim 15, **characterized in that**, the organ comprises at least one of: a brain, a heart, lungs, a liver, a stomach and kidneys.

17. The system for processing vital information according to any one of claims 1-16, **characterized in that**, displaying indication information which indicates the patient state, comprises: displaying the patient state via text or graph(s).

18. The system for processing vital information according to any one of claims 1-17, **characterized in that**, the patient data comprises vital sign data of the patient;
wherein the vital sign data comprises at least one of: electrocardiogram data, blood pressure data, pulse oximetry data, respiration data, body temperature data, cardiac output data, carbon dioxide data, movement data, video data, respiratory mechanics parameter data, hemodynamic parameter data, oxygen metabolism parameter data, electroencephalogram parameter data, bi-spectral index data, and microcirculation parameter data.

19. The system for processing vital information according to any one of claims 1-18, **characterized in that**, the patient data comprises at least one of: monitoring data and/or device data which is acquired by a ventilator device, monitoring data and/or device data which is acquired by an anesthesia machine, monitoring data and/or device data which is acquired by an infusion pump device, medical condition data, test data, and examination data.

20. The system for processing vital information according to claim 19, **characterized in that**, the medical condition data comprises at least one of: basic patient information data, disease diagnosis data, treatment data, nursing data, and electronic medical record data;
the test data comprises at least one of: routine blood test data, liver function test data, kidney function test data, thyroid test data, urine test data, immune test data, coagulation test data, blood gas test data, routine stool test data, tumor marker test data;
the examination data comprises at least one of: DR imaging data, CT imaging data, MRI imaging data, PET imaging data, ultrasound imaging data, scale data, and physical examination data.

21. The system for processing vital information according to any one of claims 1-20, **characterized in that**, the patient state is a prediction result of the patient state in the future, or a determination result of the patient state at present.

22. A method for processing vital information , **characterized in that**, comprising:
obtaining patient data of a patient;
analyzing the patient data, so as to determine a patient state of the patient, wherein the patient state at least comprises a state of a physiological system of the patient; the physiological system of the patient comprises at least one of: a nervous system, a circulatory system, and a respiratory system, of the patient;
analyzing the patient data, so as to determine one or more target rules which are satisfied by the patient data; and
controlling a display to display indication information which indicates the patient state, and to display at least a portion of the one or more target rules; wherein the indication information which indicates the patient state at least comprises an overall assessment of the state of the physiological system of the patient; wherein
when the physiological system of the patient comprises the nervous system of the patient, the one or more target rules comprise an indicator which is related to a cranial nerve;
when the physiological system of the patient comprises the circulatory system of the patient, the one or more target rules comprise an indicator which is related to hemodynamics or perfusion;
when the physiological system of the patient comprises the respiratory system of the patient, the one or more target rules comprise an indicator which is related to oxygenation.

23. The method for processing vital information according to claim 22, **characterized in that**, analyzing the patient data, so as to determine one or more target rules which are satisfied by the patient data, comprises:
analyzing the patient data, so as to obtain one or more analysis results; and
determining the one or more target rules which are satisfied by the one or more analysis results, according to the one or more analysis results.

24. The method for processing vital information according to claim 23, **characterized in that**, determining the one or more target rules which are satisfied by the one or more analysis results, according to the one or more analysis results, comprises:
comparing the one or more analysis results with one or more preset rules, and determining, from the one or more preset rules, the one or more target rules which are satisfied by the one or more analysis results.

25. The method for processing vital information according to any one of claims 22-24, **characterized in that**, analyzing the patient data, so as to determine one or more target rules which are satisfied by the patient data, comprises:
determining the one or more target rules which are satisfied by the patient data and correspond to the patient state.

26. The method for processing vital information according to claim 25, **characterized in that**, analyzing the patient data, so as to determine a patient state of the patient, comprises:
determining the patient state of the patient, according to corresponding relationship(s) between the one or more target rules and preset patient state(s).

27. The method for processing vital information according to any one of claims 22-26, **characterized in that**, analyzing the patient data, so as to determine a patient state of the patient, comprises:
determining the patient state of the patient, according to the patient data and a machine learning model for determining patient state.

28. A method for processing vital information, **characterized in that**, comprising:
obtaining patient data of a patient;
analyzing the patient data, so as to determine a patient state of the patient and to determine at least a portion of information which is extracted from the patient data and used to determine the patient state; wherein the patient state at least comprises a state of a physiological system of the patient; the physiological system of the patient comprises at least one of: a nervous system, a circulatory system, and a respiratory system, of the patient; and
displaying indication information which indicates the patient state, and displaying at least a portion of the information which is extracted from the patient data; wherein the indication information which indicates the patient state at least comprises an overall assessment of the state of the physiological system of the patient; wherein
when the physiological system of the patient comprises the nervous system of the patient, the information which is extracted from the patient data comprises an indicator which is related to a cranial nerve;
when the physiological system of the patient comprises the circulatory system of the patient, the information which is extracted from the patient data comprises an indicator which is related to hemodynamics or perfusion;
when the physiological system of the patient comprises the respiratory system of the patient, the information which is extracted from the patient data comprises an indicator which is related to oxygenation.

29. The method for processing vital information according to claim 28, **characterized in that**, analyzing the patient data, so as to determine a patient state of the patient and to determine at least a portion of information which is extracted from the patient data and used to determine the patient state, comprises:
extracting information from the patient data, and determining one or more target rules which are satisfied by said information; and
determining one or more patient states of the patient which correspond to the one or more target rules, according to corresponding relationship(s) between the target rule(s) and preset patient state(s).

30. The method for processing vital information according to claim 28, **characterized in that**, analyzing the patient data, so as to determine a patient state of the patient and to determine at least a portion of information which is extracted from the patient data and used to determine the patient state, comprises:
inputting the patient data into a machine learning model for determining patient state, so as to obtain the patient state of the patient which is outputted from the machine learning model, and determining the information which is extracted from the patient data and used to determine the patient state by the machine learning model.

31. The method for processing vital information according to any one of claims 28-30, **characterized in that**, the information which is extracted from the patient data comprises at least one of: a parameter value, an event, a result from secondary processing of parameter value, a result from secondary processing of event, and a statistical graph of the patient data.

32. The method for processing vital information according to claim 28, **characterized in that**, further comprising:
determining contribution rates of different information which is extracted from the patient data to the patient state; and
displaying the contribution rates.

33. A system for processing vital information, **characterized in that**, comprising a memory, a processor, and a display; wherein the memory is configured to store an executable program, and the processor is configured to execute the executable program, so as to enable the processor to perform following operations:
obtaining multiple types of patient data of a patient;
analyzing the patient data, so as to determine one or more target rules which are satisfied by the patient data;
determining a patient state of the patient which corresponds to the one or more target rules; and
controlling the display to display indication information which indicates the patient state, and to display at least a portion of the one or more target rules.

34. The system for processing vital information according to claim 33, **characterized in that**, the memory is further configured to store one or more preset rules;
analyzing the patient data, so as to determine one or more target rules which are satisfied by the patient data, comprises:
analyzing the patient data, so as to obtain one or more analysis results;
determining whether the one or more analysis results satisfy the one or more preset rules, and using preset rule(s) which is(are) satisfied as the one or more target rules.

35. The system for processing vital information according to claim 34, **characterized in that**, the memory is further configured to store corresponding relationship(s) between the preset rule(s) and preset patient state(s);
determining a patient state of the patient which corresponds to the one or more target rules, comprises:
determining the patient state of the patient which corresponds to the one or more target rules, according to said corresponding relationship(s).

36. The system for processing vital information according to claim 34 or claim 35, **characterized in that**, each preset rule comprises a preset condition for single analysis result, and/or, each preset rule comprises preset condition(s) for at least two analysis results.

37. The system for processing vital information according to any one of claims 34-36, **characterized in that**, analyzing the patient data, so as to obtain one or more analysis results, comprises:
analyzing a single type of the patient data, so as to obtain the analysis result(s); and/or
analyzing at least two types of the patient data, so as to obtain the analysis result(s).

38. The system for processing vital information according to any one of claims 34-37, **characterized in that**, the analysis result comprises at least one of: a parameter value, an event, a result from secondary processing of parameter value, a result from secondary processing of event.

39. The system for processing vital information according to claim 38, **characterized in that**, the event comprises at least one of: an alarm for exceeding a threshold, an abnormal event, and a clinical event.

40. The system for processing vital information according to claim 39, **characterized in that**, determining a patient state of the patient which corresponds to the one or more target rules, comprises:
using the patient data and a first machine learning model for determining patient state to determine the patient state of the patient which corresponds to the one or more target rules.

41. The system for processing vital information according to any one of claims 33-40, **characterized in that**, the processor is further configured to perform following operations:
determining a state level which represents a severity of the patient state, and
controlling the display to display indication information which indicates the state level.

42. The system for processing vital information according to any one of claims 33-41, **characterized in that**, the processor is further configured to perform following operations:
determining a physiological reason which results in the patient state according to the patient data; and
controlling the display to display indication information which indicates the physiological reason.

43. The system for processing vital information according to claim 42, **characterized in that**, the memory is further configured to store one or more preset rules, and corresponding relationship(s) between the preset rule(s) and preset physiological reason(s);
determining a physiological reason which results in the patient state according to the patient data, comprises:
analyzing the patient data, so as to obtain one or more analysis results;
determining whether the one or more analysis results satisfy the one or more preset rules, and using preset rule(s) which is(are) satisfied as the one or more target rules;
determining the physiological reason which results in the patient state according to said corresponding relationship(s).

44. The system for processing vital information according to claim 43, **characterized in that**, the processor is further configured to perform a following operation:
controlling the display to associatively display at least a portion of the one or more target rules, and the physiological reason.

45. The system for processing vital information according to claim 44, **characterized in that**, determining a physiological reason which results in the patient state according to the patient data, comprises:
using the patient data and a second machine learning model for determining physiological reason to determine the physiological reason which results in the patient state.

46. The system for processing vital information according to any one of claims 33-45, **characterized in that**, the patient state of the patient comprises at least one of: an overall state of the patient, a state of a physiological system of the patient, a state of an organ of the patient, a state of a physiological part of the patient, a state of a tissue of the patient.

47. The system for processing vital information according to claim 46, **characterized in that**, the physiological system comprises at least one of: a motor system, a nervous system, an endocrine system, a circulatory system, a respiratory system, a digestive system, a urinary system and a reproductive system; and/or
the organ comprises at least one of: a brain, a heart, lungs, a liver, a stomach and kidneys.

48. The system for processing vital information according to any one of claims 33-47, **characterized in that**, displaying indication information which indicates the patient state, comprises:
displaying the patient state via text or graph(s).

49. The system for processing vital information according to claim 48, **characterized in that**, the graph(s) correspond(s) to one or more of an overall state of the patient, a state of a physiological system of the patient, a state of an organ of the patient, a state of a physiological part of the patient, a state of a tissue of the patient.

50. The system for processing vital information according to claim 48 or claim 49, **characterized in that**, displaying the patient state comprises: presenting the patient state via marking the graph(s) with at least one of symbol information, color information and text information, which information is located on or near the graph(s).

51. The system for processing vital information according to any one of claims 33-50, **characterized in that**, the patient data comprises vital sign data of the patient;
wherein the vital sign data comprises at least one of: electrocardiogram data, blood pressure data, pulse oximetry data, respiration data, body temperature data, cardiac output data, carbon dioxide data, movement data, video data, respiratory mechanics parameter data, hemodynamic parameter data, oxygen metabolism parameter data, electroencephalogram parameter data, bi-spectral index data, and microcirculation parameter data.

52. The system for processing vital information according to any one of claims 33-51, **characterized in that**, the patient data comprises at least one of: monitoring data and/or device data which is acquired by a ventilator device, monitoring data and/or device data which is acquired by an anesthesia machine, monitoring data and/or device data which is acquired by an infusion pump device, medical condition data, test data, and examination data.

53. The system for processing vital information according to claim 52, **characterized in that**, the medical condition data comprises at least one of: basic patient information data, disease diagnosis data, treatment data, nursing data, and electronic medical record data;
the test data comprises at least one of: routine blood test data, liver function test data, kidney function test data, thyroid test data, urine test data, immune test data, coagulation test data, blood gas test data, routine stool test data, tumor marker test data;
the examination data comprises at least one of: DR imaging data, CT imaging data, MRI imaging data, PET imaging data, ultrasound imaging data, scale data, and physical examination data.

54. The system for processing vital information according to any one of claims 33-53, **characterized in that**, the patient state is a prediction result of the patient state in the future, or a determination result of the patient state at present.

55. A method for processing vital information, **characterized in that**, comprising:
obtaining multiple types of patient data of a patient;
analyzing the patient data, so as to determine one or more target rules which are satisfied by the patient data;
analyzing the patient data, so as to determine a patient state of the patient; and
displaying indication information which indicates the patient state, and displaying at least a portion of the one or more target rules.

56. The method for processing vital information according to claim 55, **characterized in that**, determining one or more target rules which are satisfied by the patient data, comprises:
analyzing the patient data, so as to obtain one or more analysis results;
determining whether the one or more analysis results satisfy one or more preset rules, and using preset rule(s) which is(are) satisfied as the one or more target rules.

57. The method for processing vital information according to claim 56, **characterized in that**, determining a patient state of the patient which corresponds to the one or more target rules, comprises:
determining the patient state of the patient which corresponds to the one or more target rules, according to corresponding relationship(s) between the preset rule(s) and preset patient state(s).

58. The system for processing vital information according to claim 56 or claim 57, **characterized in that**, each preset rule comprises a preset condition for single analysis result, and/or, each preset rule comprises preset condition(s) for at least two analysis results.

59. The system for processing vital information according to any one of claims 56-58, **characterized in that**, analyzing the patient data, so as to obtain one or more analysis results, comprises:
analyzing a single type of the patient data, so as to obtain the analysis result(s); and/or
analyzing at least two types of the patient data, so as to obtain the analysis result(s).

60. A method for processing vital information, **characterized in that**, comprising:
obtaining multiple types of patient data of a patient;
analyzing the patient data, so as to determine a patient state of the patient;
analyzing the patient data, so as to determine one or more target rules which are satisfied by the patient data; and
displaying indication information which indicates the patient state, and displaying at least a portion of the one or more target rules.

61. The method for processing vital information according to claim 60, **characterized in that**, analyzing the patient data, so as to determine one or more target rules which are satisfied by the patient data, comprises:
analyzing the patient data, so as to obtain one or more analysis results; and
determining the one or more target rules which are satisfied by the one or more analysis results, according to the one or more analysis results.

62. The method for processing vital information according to claim 61, **characterized in that**, determining the one or more target rules which are satisfied by the one or more analysis results, comprises:
comparing the one or more analysis results with one or more preset rules, and determining, from the one or more preset rules, the one or more target rules which are satisfied by the one or more analysis results.

63. The method for processing vital information according to any one of claims 60-62, **characterized in that**, analyzing the patient data, so as to determine one or more target rules which are satisfied by the patient data, comprises:
determining the one or more target rules which are satisfied by the patient data and correspond to the patient state.

64. The method for processing vital information according to claim 63, **characterized in that**, analyzing the patient data, so as to determine a patient state of the patient, comprises:
determining the patient state of the patient, according to corresponding relationship(s) between the one or more target rules and preset patient state(s).

65. The method for processing vital information according to any one of claims 60-64, **characterized in that**, analyzing the patient data, so as to determine a patient state of the patient, comprises:
determining the patient state of the patient, according to the patient data and a machine learning model for determining patient state.

66. A method for processing vital information, **characterized in that**, comprising:
obtaining multiple types of patient data of a patient;
analyzing the patient data, so as to determine a patient state of the patient and to determine at least a portion of information which is extracted from the patient data and used to determine the patient state; and
displaying indication information which indicates the patient state, and displaying at least a portion of the information which is extracted from the patient data.

67. The method for processing vital information according to claim 66, **characterized in that**, analyzing the patient data, so as to determine a patient state of the patient and to determine at least a portion of information which is extracted from the patient data and used to determine the patient state, comprises:
extracting information from the patient data, and determining one or more target rules which are satisfied by said information; and
determining one or more patient states of the patient which correspond to the one or more target rules, according to corresponding relationship(s) between the target rule(s) and preset patient state(s).

68. The method for processing vital information according to claim 67, **characterized in that**, analyzing the patient data, so as to determine a patient state of the patient and to determine at least a portion of information which is extracted from the patient data and used to determine the patient state, comprises:
inputting the patient data into a machine learning model for determining patient state, so as to obtain the patient state of the patient which is outputted from the machine learning model, and determining the information which is extracted from the patient data and used to determine the patient state by the machine learning model.

69. The method for processing vital information according to any one of claims 66-68, **characterized in that**, the information which is extracted from the patient data comprises at least one of: a parameter value, an event, a result from secondary processing of parameter value, a result from secondary processing of event, and a statistical graph of the patient data.

70. The method for processing vital information according to any one of claims 66-69, **characterized in that**, further comprising:
determining contribution rates of different information which is extracted from the patient data to the patient state; and
displaying the contribution rates.

71. The method for processing vital information according to any one of claims 55-70, **characterized in that**, the patient state comprises at least one of: an overall state of the patient, a state of a physiological method of the patient, a state of an organ of the patient, a state of a physiological part of the patient, a state of a tissue of the patient.

72. The system for processing vital information according to claim 71, **characterized in that**, the physiological system comprises at least one of: a nervous system, a respiratory system, a circulatory system, a motor system, an endocrine system, a digestive system, a urinary system and a reproductive system; and/or
the organ comprises at least one of: a brain, a heart, lungs, a liver, a stomach and kidneys.

73. The system for processing vital information according to any one of claim 55, 60 or 66, **characterized in that**:
when a physiological system of the patient comprises a nervous system of the patient, the one or more target rules comprise an indicator which is related to a cranial nerve;
when a physiological system of the patient comprises a circulatory system of the patient, the one or more target rules comprise an indicator which is related to hemodynamics or perfusion;
when a physiological system of the patient comprises a respiratory system of the patient, the one or more target rules comprise an indicator which is related to oxygenation.

74. The method for processing vital information according to any one of claims 55-73, **characterized in that**, further comprising:
determining a physiological reason which results in the patient state according to the patient data; and
displaying indication information which indicates the physiological reason.

75. The method for processing vital information according to any one of claims 55-74, **characterized in that**, displaying indication information which indicates the patient state, comprises:
displaying the patient state via text or graph(s).

76. The method for processing vital information according to any one of claims 55-75, **characterized in that**, the patient data comprises vital sign data of the patient;
wherein the vital sign data comprises at least one of: electrocardiogram data, blood pressure data, pulse oximetry data, respiration data, body temperature data, cardiac output data, carbon dioxide data, movement data, video data, respiratory mechanics parameter data, hemodynamic parameter data, oxygen metabolism parameter data, electroencephalogram parameter data, bi-spectral index data, and microcirculation parameter data.

77. The method for processing vital information according to any one of claims 55-76, **characterized in that**, the patient data comprises at least one of: monitoring data and/or device data which is acquired by a ventilator device, monitoring data and/or device data which is acquired by an anesthesia machine, monitoring data and/or device data which is acquired by an infusion pump device, medical condition data, test data, and examination data.

78. The method for processing vital information according to claim 77, **characterized in that**, the medical condition data comprises at least one of: basic patient information data, disease diagnosis data, treatment data, nursing data, and electronic medical record data;
the test data comprises at least one of: routine blood test data, liver function test data, kidney function test data, thyroid test data, urine test data, immune test data, coagulation test data, blood gas test data, routine stool test data, tumor marker test data;
the examination data comprises at least one of: DR imaging data, CT imaging data, MRI imaging data, PET imaging data, ultrasound imaging data, scale data, and physical examination data.

79. The method for processing vital information according to any one of claims 55-78, **characterized in that**, the patient state is a prediction result of the patient state in the future, or a determination result of the patient state at present.
